(19) Europäisches Patentamt
European Patent Office
Office européen des brevets

(11) EP 1 409 662 B1

(12) # EUROPÄISCHE PATENTSCHRIFT

(45) Veröffentlichungstag und Bekanntmachung des Hinweises auf die Patenterteilung:
**01.02.2006 Patentblatt 2006/05**

(21) Anmeldenummer: **02784846.4**

(22) Anmeldetag: **06.07.2002**

(51) Int Cl.:
***C12N 9/88*** *(2006.01)* ***C12N 9/02*** *(2006.01)*
***C12N 15/82*** *(2006.01)*

(86) Internationale Anmeldenummer:
**PCT/EP2002/007555**

(87) Internationale Veröffentlichungsnummer:
**WO 2003/006647 (23.01.2003 Gazette 2003/04)**

(54) **ALLENOXIDSYNTHASE UND DIVINYLETHER SYNTHASE AUS DER CYP74-ENZYMFAMILIE ISOLIERT AUS PHYSCOMITRELLA PATENS SOWIE DIE SIE KODIERENDEN NUKLEOTIDSEQUENZEN UND VERFAHREN ZUR HERSTELLUNG VON PATHOGENRESISTENTEN PFLANZEN**

ALLENE OXIDE SYNTHASE AND DIVINYL ETHER SYNTHASE FROM THE CYP74-ENZYME FAMILY ISOLATED OUT OF PHYSCOMITRELLA PATENS, THE NUCLEOTIDE SEQUENCES THAT CODE THESE SYNTHASES, AND METHOD FOR PRODUCING PATHOGEN-RESISTANT PLANTS

ALLENE OXYDE SYNTHASE ET DIVINYL ETHER SYNTHASE DE LA FAMILLE D'ENZYMES CYP74 ISOLEES A PARTIR DE PHYSCOMITRELLA PATENS, SEQUENCES NUCLEOTIDIQUES CODANT POUR CES ENZYMES, AINSI QUE PROCEDE POUR LA PRODUCTION DE PLANTES RESISTANTES AUX PATHOGENES

(84) Benannte Vertragsstaaten:
**AT BE BG CH CY CZ DE DK EE ES FI FR GB GR IE IT LI LU MC NL PT SE SK TR**

(30) Priorität: **12.07.2001 DE 10133343**
**06.05.2002 DE 10220115**

(43) Veröffentlichungstag der Anmeldung:
**21.04.2004 Patentblatt 2004/17**

(73) Patentinhaber: **BASF Plant Science GmbH**
**67056 Ludwigshafen (DE)**

(72) Erfinder:
- **FEUSSNER, Ivo**
**37085 Göttingen (DE)**
- **STUMPE, Michael**
**06114 Halle (DE)**

(74) Vertreter: **Fitzner, Uwe et al**
**Dres. Fitzner & Münch**
**Rechts- und Patentanwälte**
**Hauser Ring 10**
**40878 Ratingen (DE)**

(56) Entgegenhaltungen:
**WO-A-01/38484** **WO-A-02/29018**

- **SONG WEN-CHAO ET AL: "Molecular cloning of an allene oxide synthase: A cytochrome P450 specialized for the metabolism of fatty acid hydroperoxides" PROCEEDINGS OF THE NATIONAL ACADEMY OF SCIENCES OF USA, NATIONAL ACADEMY OF SCIENCE. WASHINGTON, US, Bd. 90, Nr. 18, September 1993 (1993-09), Seiten 8519-8523, XP002136819 ISSN: 0027-8424**
- **HARMS KARSTEN ET AL: "Expression of a flax allene oxide synthase cDNA leads to increased endogenous jasmonic acid (JA) levels in transgenic potato plants but not to a corresponding activation of JA-responding genes" PLANT CELL, AMERICAN SOCIETY OF PLANT PHYSIOLOGISTS, ROCKVILLE, MD, US, Bd. 7, Nr. 10, Oktober 1995 (1995-10), Seiten 1645-1654, XP002136820 ISSN: 1040-4651**
- **WANG CUNXI ET AL: "Overexpression of a cytoplasm-localized allene oxide synthase promotes the wound-induced accumulation of jasmonic acid in transgenic tobacco." PLANT MOLECULAR BIOLOGY, Bd. 40, Nr. 5, Juli 1999 (1999-07), Seiten 783-793, XP002215177 ISSN: 0167-4412**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

(Forts. nächste Seite)

- PAN ZHIQIANG ET AL: "The major protein of guayule rubber particles is a cytochrome P450: Characterization based on cDNA cloning and spectroscopic analysis of the solubilized enzyme and its reaction products." JOURNAL OF BIOLOGICAL CHEMISTRY, Bd. 270, Nr. 15, 1995, Seiten 8487-8494, XP002215178 ISSN: 0021-9258
- DATABASE EMBL [Online] EBI; 16. April 2000 (2000-04-16) SHELDRICK B.: "Lycopersicon esculentum mRNA for allene oxide synthase (aos gene)" Database accession no. AJ271093 XP002215181
- MAUCHER HELMUT ET AL: "Allene oxide synthases of barley (Hordeum vulgare cv. Salome): Tissue specific regulation in seedling development." PLANT JOURNAL., Bd. 21, Nr. 2, Januar 2000 (2000-01), Seiten 199-213, XP002215179 ISSN: 0960-7412 in der Anmeldung erwähnt
- ITOH ET AL: "Molecular cloning of divinyl ether synthase. Identification as a CYP74 cytochrome P-450" JOURNAL OF BIOLOGICAL CHEMISTRY, AMERICAN SOCIETY OF BIOLOGICAL CHEMISTS, BALTIMORE, MD, US, Bd. 276, Nr. 5, 2. Februar 2001 (2001-02-02), Seiten 3620-3627, XP002204173 ISSN: 0021-9258
- DATABASE EMBL [Online] EBI; 3. Februar 2001 (2001-02-03) FEUSSNER I.: "Solanum Tuberosum mRNA for divinyl ether synthase (des gene)" Database accession no. AJ309541 XP002215182 -& STUMPE M. ET AL.: "A pathogen-inducible divinyl ether synthase (CYP74D) from elicitor treated potato suspension cells" FEBS LETTERS, Bd. 507, 19. Oktober 2001 (2001-10-19), Seiten 371-376, XP004320956
- ZANK T K ET AL: "Cloning and functional expression of the first plant fatty acid elongase specific for DELTA6-polyunsaturated fatty acids" BIOCHEMICAL SOCIETY TRANSACTIONS, COLCHESTER, ESSEX, GB, Bd. 28, Nr. 6, Dezember 2000 (2000-12), Seiten 654-658, XP002174836 ISSN: 0300-5127
- GIRKE THOMAS ET AL: "Identification of a novel DELTA6-acyl-group desaturase by targeted gene disruption in Physcomitrella patens." PLANT JOURNAL, Bd. 15, Nr. 1, Juli 1998 (1998-07), Seiten 39-48, XP002215180 ISSN: 0960-7412

## Beschreibung

Die vorliegende Erfindung betrifft Enzyme der Cytochrom-P450-Klasse und die Isolierung der entsprechend kodierenden Nukleotidsequenzen sowie deren Einsatz in einem Verfahren zur Herstellung pathogenresistenter Pflanzenzellen und deren Nachkommen.

Zu der Klasse der Cytochrom P450-Enzyme gehören die Enzyme Allenoxid-Synthase (AOS), die Hydroperoxid-Lyase (HPL) und die Divinylether-Synthase (DES). Sie bilden eine eigene Unterfamilie mit dem Namen *CYP74*. Aufgrund der Vielzahl von Cytochrom P450-Enzymen wurde eine Nomenklatur entwickelt, die jedem Protein dieser Klasse aufgrund der Primärstruktur eine spezifische Familie und Unterfamilie zuordnet So bilden alle AOS die eigene Unterfamilie *CYP74A,* unter *CYP74B* sind die 13-HPL, unter *CYP74C* die 9/13-HPL und unter *CYP74D* die 9-DES zusammengefasst (Feussner et al.; 2001, *Trends Plant Sci. 6*, 268-273). Proteine der gleichen Unterfamilie werden chronologisch durchnummeriert.

CYP74-Enzyme sind Monooxygenasen mit einem Häm-Molekül als prosthetischer Gruppe. Obwohl sie als prosthetische Gruppe auch eine Protoporphyrin IX Gruppe (Häm b) tragen, besitzten sie nur eine sehr geringe Affinität zu CO (Matsui, 1998, *Belgian Journal of Botany. 131, 50*-62).
Sie sind wichtige Enzyme im Metabolismus von Polyenfettsäuren, dem sogenannten Lipoxygenase (LOX)-Reaktionsweg (Feussner and Wastemack, 1998, *Fett/Lipid. 100*, 146-152).

LOXs sind Dioxygenasen, bei denen das Eisen im katalytischen Zentrum an Aminosäureseitenketten gebunden ist (Brash, 1999, *J. Biol. Chem. 274*, 23679-23682). Sie katalysieren den Einbau von molekularem Sauerstoff in das (1Z,4Z)-Pentadiensystem von mehrfach ungesättigten Fettsäuren. In Pflanzen sind das vor allem Linol- und $\alpha$-Linolensäure. Als Produkte können in Abhängigkeit von der Regioselektivität der eingesetzten LOX zwei verschiedene Positionsisomere von Hydroxyperoxiden, nämlich (9*S*)-Isomere oder (13*S*)-Isomere entstehen. Z.B. wird aus $\alpha$-Linolensäure (13*S*,9*Z*,11*E*,15*Z*)-13-Hydroperoxy-9,11,15-octadecatriensäure (13S-HPOTE) und aus Linolsäure (13*S*,9*Z*,11*E*)-13-Hydroperoxy-9,11-octadecadiensäure (13*S*-HPODE).

In Pflanzen werden diese Hydroperoxide schnell durch eine Vielzahl von Enzymen weiter umgesetzt. Zur Zeit sind sieben verschiedene Enzymfamilien im Pflanzenreich bekannt, die Hydroperoxide umsetzen und damit um LOX-Produkte konkurrieren: die Allenoxid-Synthase (AOS)-Reaktion, die Hydroperoxid-Lyase (HPL)-Reaktion, die Divinylether-Synthase (DES)-Reaktion, die Reduktase-Reaktion, die Peroxygenase-Reaktion, die Epoxyalkohol-Synthase (EAS)-Reaktion und die LOX-Reaktion selbst (Feussner et al.; 2001, *Trends Plant Sci. 6*, 268-273). Bei der Umsetzung von 13-HPOTE in Gegenwart des Enzyms Allenoxid-Cyclase (AOC) beobachtet man eine Zyklisierung zu 12-Oxophytodiensäure (12-Oxo-PDA) (Ziegler et al.; 2000, *J. Biol. Chem. 275,* 19132-8), die wiederum die Vorstufe der Jasmonsäure ist, die den Pflanzenhormonen zugeordnet wird.

Die AOS (EC4.2.1.92; CYP74A) ist das erste beschriebene *CYP74*-Enzym und wurde erstmalig als homogenes Protein aus Flax isoliert (Song and Brash, 1991, *Sience. 253*, 781-784). Sie katalysiert die Umsetzung zu einem instabilen Allenoxid, welches zu den entsprechenden $\alpha$- und $\gamma$-Ketolen in Gegenwart von Wasser zerfallen kann. Die AOS ist an der Biosynthese von Jasmonsäure (Vick and Zimmerman, 1983, *Biochem. Biophys. Res. Commun. 111,* 470-7) beteiligt Jasmonsäure selbst ist an der Induktion der Transkription spezifischer mRNA und der Regulation der Translation von Jasmonat-induzierten Proteinen (JIP), wie LOX, AOS und Proteinase-Inhibitoren beteiligt Dies macht Jasmonsäure zu einem wichtigen Signaistoff bei der pflanzlichen Stressantwort (Wastemack and Parthier, 1997, *Trends Plant Sci. 2*, 302-307). Eine Beteiligung an Prozessen der Wachstumsregulafion und Seneszenzförderung wird ebenfalls diskutiert (Sembdner and Parthier, 1993, *Annu. Rev. Plant Physiol. Plant Mol. Biol. 44*, 569-589).
Zahlreiche AOS wurden schon kloniert und in *E. coli* funktionell exprimiert, darunter die AOS aus *Arabidopsis thaliana, Lycopersicon esculentum, Linum usitatissimum* und *Hordeum vulgare.* Abgesehen von den AOS der Gerste besitzen alle bisher klonierten AOS eine Substratspezifität für (13*S*)-Hydroperoxide (Maucher et al.; 2000, *Plant J. 21,* 199-213).

Die HPL (*CYP74B* und *C*) spaltet die Hydroperoxide zu (3Z)-Aldehyden und $\omega$-Oxo-Säuren (Matsui, 1998, *Belgian Journal of Botany. 131,* 50-62). Noch bevor das Enyzm selbst entdeckt wurde, waren die Reaktionsprodukte der HPL als „Blattafdehyde" bekannt, die für den charakteristischen Geruch von Pflanzen und Früchten mit verantwortlich (Hatanaka, 1996, *Food Rev. Int. 12,* 303-350) sind. Im Falle von 13-HPOTE als Substrat entsteht (3Z)-Hexenal und (9*Z*)-12-Oxo-9-Dodecensäure, die zu (10*E*)-12-Oxo-10-Dodecensäure (Traumatin) isomerisiert, welches als Wundhormon diskutiert wird. Eine Funktion als pflanzlicher Botenstoff wird ebenso diskutiert (Bate and Rothstein, 1998, *Plant J. 16,* 561-569). Traumatin kann noch weiter zur Traumatinsäure oxidiert werden, das ebenfalls an der Wundantwort von Pflanzen beteiligt zu sein scheint (Zimmerman and Vick, 1970, *Plant Physiol. 46*, 445-453). Kloniert und in *E. coli* als aktives Protein exprimiert wurden unter anderem die HPL aus *A. thaliana, Cucumis sativus, Medicago sativus* und *L. esculentum.* Auch hier zeigt sich eine Substratspezifität für (13S)-Hydroperoxide bei den meisten Enzymen. Lediglich je eine HPL aus Gurke und aus Melone sind ohne Substratspezifität und werden daher als 9/13-HPL bezeichnet (McIntyre et al.; 1999, *J. Biol. Chem. 274*, 25189-25192; Matsui et al.; 2000, *FEBS Left. 481,* 183-188). Werden beide Sequenzen auf ihre Verwandtschaft zu anderen Mitgliedern der *CYP74*-Familie untersucht, so zeigt sich ein höherer Grad der Homologie zu AOS als zu den 13-HPL. Daher werden diese Enzyme in eine eigene Subfamilie, *CYP74C,* eingeordnet (Matsui et al.; 2000, *FEBS Lett. 481*, 183-188). Bei der HPL von *Arabidopsis* konnte gezeigt werden, daß sie durch Verwundung induziert wird.

Die DES *(CYP74D)* katalysiert die Bildung von Divinylethern, die fungizide Wirkung aufweisen (Weber et al. 1999). Ebenso wird diskutiert, daß die Divinylether, wie die Aldehyde im Falle der HPL-Produkte, bei der Abwehr von pathogenen Pilzen und Bakterien eine Rolle spielen (Weber et al.; 1999, *Plant Cell. 11*, 485-493; Göbel et al.; 2001, *J. Biol. Chem. 276,* 6267-6273). Die erste DES aus *L. esculentum* wurde von Itoh and Howe 2001 kloniert Dabei zeigte sich, daß sie auf Sequenzebene hohe Homologien zur AOS und HPL besitzt, so daß sie auch zur Cytochrom P450-Klasse, Subfamilie *CYP74D,* gerechnet wird. Darüber hinaus ist die DES in der Gruppe der *CYP74* einzigartig darin, daß sie als einzige hoch spezifisch für *9S*-Hydroperoxide ist (Itoh and Howe, 2001, *J. Biol. Chem. 276*, 3620-3627).

In der Natur sind P450-Enzyme vielfach in die Biosynthese und den Metabolismus von zahlreichen endogenen Stoffen involviert. Eine besondere Rolle spielt ihre Beteiligung an der Detoxifikation von Xenobiotika. Pflanzliche P450-Enzyme sind ferner an der Biosynthese von Wundsignalen (Jasmonsäure, Salicylsäure, Traumatin) und Hormonen (Gibberelline, Brassinosteroide) beteiligt

Auslöser von Wundsignalen in Pflanzen und nachfolgende Signaltransduktionskaskaden sind vielgestaltig. Sie können durch Beschädigung oder Verletzung der Pflanze ausgelöst werden, aber auch (künstlich) durch von außen zugeführte chemische Verbindungen induziert werden.

Darüber hinaus ist aber insbesondere das Auftreten von Pflanzenkrankheiten und vor allem die Steigerung der pflanzlichen Abwehr gegen solche Krankheitserreger agrarwirtschaftfich von enormer Relvanz. Dabei kann die pflanzliche Antwort auf einen Krankheitserreger auf einem vollkommen anderen Weg erfolgen, als dies bei der Stessabwehr durch Verwundung der Fall ist. Das Auftreten von Pflanzenkrankheiten, beispielsweise hervorgerufen durch Viren, Bakterien oder Pilze, führt in der Regel zu erheblichen Schädigungen oder sogar zum Ausfall der ganzen Pflanze, verbunden mit einer drastisch verringerten Menge oder Qualität des Emtegutes.

Um die Ertragsverluste auf ein wirtschaftlich vertretbares Maß zu begrenzen, sind Pflanzenschutzmaßnahmen unabdingbar. Insbesondere wünschenswert wäre es, wenn auch ohne den Einsatz von Chemikalien, die eine zusätzliche Belastung für Boden, Grundwasser sowie den Anwender darstellen, die Abwehr von Pflanzen gegenüber Krankheitserregern und/oder Schädlingen zu verbessern.

Die vorliegende Erfindung hat sich daher zur Aufgabe gemacht, transgene Pflanzenzellen, Pflanzen und deren Nachkommen zur Verfügung zu stellen, die über eine erhöhte Pathogenresistenz verfügen sowie entsprechend verbesserte Verfahren zu deren Herstellung bereitzustellen.

Diese Aufgabe wird gelöst, durch eine Allenoxid-Synthase mit einer Aminosäuresequenz gemäß SEQ ID No. 2 und/oder eine Divinylether-Synthase mit einer Aminosäuresequenz gemäß SEQ ID No. 4.

Die beiden zuvor genannten Enzyme gehören zur Familie der CYP74-Enzyme. Eine gesteigerte spezifische Aktivtät dieser CYP74-Enzyme, einzeln oder in Kombination, mit Bezug auf die in einer Pflanzenzelle endogen vorliegende spezifische Aktivität dieser Enzyme führt in einer Pflanzenzelle zu einer gesteigerten Resistenz gegenüber Krankheitserregern.

Dabei bewirkt eine erfindungsgemäß erhöhte spezifische Aktivtät der CYP-Enzyme gegenüber der in Pflanzenzellen oder Pflanzen endogen vorliegenden spezifischen Aktivität eine Steigerung der Resistenz der Pflanzenzellen oder Pflanzen gegenüber Krankheitserregern um 20-90%, bevorzugt um 30-80% und besonders bevorzugt um 40-70%. Die Resistenz der Pflanze wird dabei über die Abnahme der Penetrationsfrequenz ermittelt.

Unter Penetrationsfrequenz ist erfindungsgemäß die Anzahl der Infektionsstellen mit erfolgreich penetrierten Epidermiszelle zu verstehen, dividiert durch die Gesamtzahl der Infektionsstellen.

Die erfindungsgemäßen CYP74-Enzyme, einzeln oder in Kombination, bewirken in vorteilhafter Weise in Pflanzen eine erhöhte Resistenz gegenüber Krankheitserregern, wie z.B. biotrophen Pilzen. Bevorzugt bewirkt eine erhöhte Konzentration (erhöhte spezifische Aktivität) an erfindungsgemäßen CYP74-Protein/en eine erhöhte Resistenz gegenüber Mehltaupilzen, besonders bevorzugt Blumeria graminis f. sp. hordei oder f. sp. tritici. Eine erhöhte Resistenz gegenüber weiteren Pflanzenpathogenen ist dadurch jedoch nicht ausgeschlossen.

Weitere Beispiele solcher Pflanzenpathogene sind Pythium spec., Albugo spec., Rhizoctonia solani, Peronospora parasitica, Erysiphe crucifearum, E. cichoreacearum, Alternaria brassicicola, Botrytis cinerea, Sclerotium rolfsii, Sclerotinia sclerotium, Fusarium oxysporum, F. culmorum, F. graminearum, F. nivale, Phytophtora infestans oder Pseudomonas syringae.

Bemerkenswert ist, daß die zuvor genannten erfindungsgemäßen CYP74-Enyzme ein sehr viel breiteres Substratspektrum aufweisen als bislang bekannte CYP-Enzyme dieser Klasse. Die erfindungsgemäßen CYP74-Enyzme können sowohl 9-HPOD/TE als auch 13-HPOD/TE als Substrat umsetzen.

Erfindungsgemäß stammen die hier vorliegenden CYP74-Enzyme aus Moos oder höheren Pflanzen. Bevorzugt stammen die erfindungsgemäßen CYP74-Enzyme aus Physcomitrella patens.

Gegenstand der vorliegenden Erfindung ist auch eine isolierte Nukleotidsequenz kodierend für eine Allenoxid-Synthase der zuvor genannten Art, beteiligt an der Biosynthese von mehrfach ungesättigten Fettsäuren zur Steigerung der Resistenz von Pflanzenzellen oder Pflanzen gegenüber Krankheitserregern ausgewählt aus

a) einer Nukleotidsequenz gemäß SEQ ID No. 1,

b) einer Nukleotidsequenz, die wenigstens 70% Identität mit der in SEQ ID No. 1 gezeigten Nukleotidsequenz aufweist,

c) einer zu a) oder b) komplementären Nukleotidsequenz.

Ebenso ist erfindungsgemäß eine isolierte Nukleotidsequenz umfaßt, kodierend für eine Divinylether-Synthase der zuvor genannten Art beteiligt an der Biosynthese von mehrfach ungesättigten Fettsäuren zur Steigerung der Resistenz von Pflanzenzellen oder Pflanzen gegenüber Krankheitserregern, ausgewählt aus

a) einer Nukleotidsequenz gemäß SEQ ID No. 3,

b) einer Nukleotidsequenz, die wenigstens 70% Identität mit einer der in SEQ ID No. 3 gezeigten Nukleotidsequenz aufweist,

c) einer zu a) oder b) komplementären Nukteotidsequenz.

Unter einer isolierten Nukleinsäure ist erfindungsgemäß ein Polymer aus RNA oder DNA zu verstehen, das einzel- oder doppelsträngig sein kann und optional natürliche, chemisch synthetisierte, modifizierte oder artifizielle Nukleotide enthalten kann. Der Begriff DNA-Polymer schließt hierbei auch genomische DNA, cDNA oder Mischungen davon ein.

Unter komplementären Nukleotidsequenzen sind erfindungsgemäß DNA-oder RNA- (mRNA) Sequenzen zu verstehen, die eine Abschrift der entsprechenden Ausgangssequenz gemäß den Basenpaarungsregeln darstellt

Die erfindungsgemäßen Nukleotidsequenzen zeichnen sich ferner dadurch aus, daß sie aus Moos oder höheren Pflanzen stammt Bevorzugt stammen sie aus Physcomitrella patens.

Zur Begriffsklärung sei bemerkt,daß das durch die SEQ ID No. 1 kodierte Protein ein Cytochrom P450-Enzym der Klasse CYP74A (Allenoxid-Synthase; AOS) ist. Das durch die SEQ ID No. 3 kodierte Enzym wird, u.a. aufgrund seiner Substratspezifität, der Klasse CYP74E (Divinylether-Synthase; DES) zugeordnet.

Gegenstand der vorliegenden Erfindung ist ferner ein Genkonstrukt enthaltend eine Nukleotidsequenz gemäß SEQ ID No. 1 und/oder eine Nukleotidsequenz gemäß SEQ ID No. 3 sowie operativ damit verknüpfte regulatorische Nukleotidsequenzen.

Hierbei können die kodierenden Bereiche einzeln oder in Kombination (d.h. gemeinsam) unter der Kontrolle derselben regulatorischen Sequenzen oder verschiedener separater regulatorischer Sequenzen stehen.

Unter einer operativen Verknüpfung versteht man die sequenzielle Anordnung beispielsweise von Promotor, kodierender Sequenz, Terminator und ggf. weiterer regulatorischer Elemente derart, daß jedes der regulatorischen Elemente seine Funktion bei der Expression der kodierenden Sequenz bestimmungsgemäß erfüllen kann. Diese regulatorischen Nukleotidsequenzen können natürlichen Ursprungs sein oder durch chemische Synthese erhalten werden. Als Promotor ist grundsätzlich jeder Promotor geeignet, der die Genexpression in dem entsprechenden Wirtsorganismus steuern kann. Hierbei kann es sich erfindungsgemäß auch um einen natürlichen oder synthetisch erzeugten chemisch induzierbaren Promotor handeln, durch den die Expression der ihm unterliegenden Gene in der Wirtszelle zu einem bestimmten Zeitpunkt gesteuert werden kann. Hierzu zählen auch gewebespezifische Promotoren. Die Herstellung einer Genstruktur erfolgt durch Fusion eines geeigneten Promotors mit wenigstens einer erfindungsgemäßen Nukleotidsequenz nach gängigen Rekombinations- und Klonierungstechniken, wie sie beispielsweise in T. Maniatis, E.F. Fritsch und J. Sambrook, Molecular Cloning: A Laboratory Manual, Cold Spring Harbor Laboratury, Cold Spring Harbor, NY (1989) beschrieben sind. Für die Verbindung der DNA-Fragmente miteinander können an die Fragmente Adaptoren oder Linker angesetzt werden.

Erfindungsgemäß sind auch die den kodierenden Bereichen (Strukturgenen) vorausgehenden (5'- oder upstream) und/oder nachfolgenden (3'- oder downstream) Sequenzbereiche eingeschlossen. Insbesondere sind hierin Sequenzbereiche mit regulatorischer Funktion inbegriffen. Sie können die Transkription, die RNA-Stabilität oder das RNA processing sowie die Translation beeinflussen. Beispiele für regulatorische Sequenzen sind u. a. Promotoren, Enhancer, Operatoren, Terminatoren oder Transtationsverstärker.

In der vorliegenden Erfindung umfaßt ist außerdem ein Vektor enthaltend eine isolierte Nukleotidsequenz gemäß SEQ ID No. 1 und/oder eine isolierte Nukleotidsequenz gemäß SEQ ID No. 3 und/oder ein Genkonstrukt der zuvor genannten Art sowie zusätzliche Nukleotidsequenzen zur Selektion und/oder Replikation in einer Wirtszelle und/oder zur Intregration in das Genom einer Wirtszelle.

Erfindungsgemäß geeignete Wirtszellen können allgemein Zellen höherer Pflanzen sein. Bevorzugt sind Zellen von Nutzpflanzen, bevorzugt monocotyledonen Nutzpflanzen, besonders bevorzugt Getreide und insbesondere Gerste und/oder Weizen. Bevorzugte Zellen dicotyledoner Nutzpflanzen sind solche der Familie der Solanaceen.

Die vorliegende Erfindung umfaßt somit auch wenigstens eine transgene Pflanzenzelle, ganze Pflanze und/oder deren Nachkommen, enthaltend in replizierbarer Form eine isolierte Nukleotidsequenz gemäß SEQ 10 No. 1/ und/oder eine isolierte Nukleotidsequenz gemäß SEQ ID No. 3/ und/oder ein Genkonstrukt der zuvor genannten Art und/oder einen Vektor der zuvor genannten Art, wobei die transgene Pflanzenzeile, ganze Pflanze und/oder deren Nachkommen eine verstärkte Expression der Nukleotidsequenz kodierend für eine Allenoxid-Synthase und/oder der Nukleotidsequenz kodierend für eine Divinylether-Synthase im Vergleich zu der endogen vorliegenden Genexpression (d.h. natürlicherweise vorliegende Genexpression, wie sie z.B. in einer nicht-transformierten Pflanze oder Pflanzenzelle vorliegt) aufweist, die zu einer gesteigerten Resistenz von Pflanzen gegenüber Krankheitserregern führt.

Eine verstärkte Genexpression kann in einer erhöhten Kopienzahl der entsprechenden Nukleotidsequenz begründet sein. Oder sie beruht beispielsweise darauf, daß die kodierende Region einer Nukleotidsequenz operativ mit einer oder mehreren regulatorischen Sequenzen verknüpft ist, die eine verstärkte Initionation der Genexpression bewirken. Dies kann z.B. durch einen starken und/oder induzierbaren Promotor und/oder Enhancer und/oder andere regulatorische Sequenzen bewirkt werden.

Hinsichtlich der Kopienzahl kann in einer Variante der vorliegenden Erfindung eine oder beide der genannten Nukleotidsequenzen in 2-100, bevorzugt 5-50 und besonders bevorzugt in 2-15 Kopien vorliegen.

In den zuvor genannten erfindungsgemäßen transgenen Pflanzenzellen, Pflanzen und/oder deren Nachkommen können die erfindungsgemäßen Nukleotidsequenzen (einzein oder in Kombination) und/oder Genkonstrukte und/oder Vektoren der zuvor genannten Art extrachromosomal vorliegen und/oder stabil in das pflanzliche Genom integriert sein.

Entsprechende Vorgehensweisen und Hilfsmittel, wie Genkonstrukte oder Vektoren und geeignete Hilfsorganismen zur integration der Nukleotidsequenz in das pflanzliche Genom sind dem Fachmann geläufig und werden nicht näher ausgeführt.

Die transgene Pflanzenzelle, ganze Pflanze und/oder deren Nachkommen, welche eine Allenoxid-Synthase gemäß SEQ ID No. 2 und/oder eine Divinylether-Synthase gemäß SEQ ID No. 4 aufweist, wobei die entsprechende spezifische Aktivität der Enzyme in den transgenen Wirtssystemen gegenüber der entsprechenden endogenen Enzymaktivität (z.B. in den Wildtypzellen) erhöht ist, ist ebenfalls von der vorliegenden Erfindung umfaßt

Erfindungsgemäß handelt es sich bei der zuvor genannten transgenen Pflanzenzelle, ganzen Pflanze und/oder deren Nachkommen um eine Nutzpflanze oder deren Zellen, bevorzugt der Familie der Solanaceae oder Getreide, besonders bevorzugt Kartoffel, Gerste oder Weizen.

Die vorliegende Erfindung umfaßt auch ein Verfahren zur Steigerung der Resistenz von Pflanzenzellen oder Pflanzen gegenüber Krankheitserregern, wobei eine Nukleotidsequenz gemäß SEQ ID No. 1 und/oder eine Nukleotidsequenz gemäß SEQ ID No. 3 und/oder ein Genkonstrukt und/oder ein Vektor der zuvor erläuterten Art in replizierbarer Form in Pflanzenzellen übertragen wird und aus den so transformierten Pflanzenzellen ganze Pflanzen regeneriert werden.

Verfahren zur Herstellung solcher erfindungsgemäßen trangenen Pflanzen gehören zur gängigen Laborpraxis. In einer vorteilhaften Variante der vorliegenden Erfindung werden die Nukleotidsequenz und/oder Genkonstrukte und/oder Vektoren durch sogenanntes ‚particle bombardment' und/oder Agrobakterien-vermittelte Transformation in die Pflanze oder Pflanzenteile oder Zellen übertragen.

Ein besonderer Vorteil der erfindungsgemäß erhaltenen transgenen Pflanze und deren Nachkommen ist, daß eine Erhöhung der Kopienzahl wenigstens einer der erfindungsgemäßen Nukleotidsequenzen oder analog das Vorliegen einer erhöhten Konzentation wenigstens eines entsprechend kodierten Proteins zu einer wesentlich gesteigerten Krankheitsresistenz führt. D.h. das/die erfindungsgemäße/n Gen/e bzw. das/die kodierte/n Protein/e ist kausal an der Ausbildung der Resistenz gegenüber pathogenen Schädlingen beteiligt.

Beispiele für pathogene Schädlinge sind u.a. Blumeria graminis f. sp. hordei oder f. sp. tritici, Pythium spec., Albugo spec., Rhizoctonia solani, Peronospora parasitica, Erysiphe crucifearum, E. cichoreacearum, Alternaria brassicicola, Botrytis cinerea, Sclerotium rolfsii, Sclerotinia sclerotium, Fusarium oxysporum, F. culmorum, F. graminearum, F. nivale, Phytophtora infestans oder Pseudomonas syringae.

In einer bevorzugten Variante des erfindungsgemäßen Verfahrens werden als pflanzliche Zellen solche von Nutzpflanzen, bevorzugt der Familie der Solanaceae oder von Getreide, besonders bevorzugt Karboffel, Gerste oder Weizen eingesetzt

Gegenstand der vorliegenden Erfindung ist ferner die Verwendung einer Nukleotidsequenz gemäß SEQ ID No. 1 und/oder einer Nukleotidsequenz gemäß SEQ ID No. 3 zur Steigerung der Resistenz transgener Pflanzenzellen, ganzer Pflanzen und/oder deren Nachkommen gegenüber Krankheitserregern.

Die vorliegende Erfindung umfaßt auch die Verwendung von wenigstens einem Polypeptid gemäß SEQ ID No. 2 und/oder einem Polypeptid gemäß SEQ ID No. 3 zur Steigerung der Resistenz transgener Pflanzenzellen, ganzer Pflanzen und/oder deren Nachkommen gegenüber Krankheitserregern.

Erfindungsgemäß ist auch die Verwendung von wenigstens einem der zuvor genannten Enzyme umfaßt, wobei eine erhöhte spezifische Aktivtät wenigstens eines Enzyms gegenüber der entsprechenden endogenen spezifischen Enzym-Aktivität eine Steigerung der Resistenz der Pflanzenzellen oder Pflanzen gegenüber Krankheitserregern um 20-90%, bevorzugt um 30-80% und besonders bevorzugt um 40-70% bewirkt.

Hierbei bewirkt die Verwendung von wenigstens einem der erfindungsgemäßen Enzyme beispielsweise eine erhöhte Resistenz gegenüber Mehltaupilzen, bevorzugt Blumeria graminis f. sp. hordei oder f. sp. tritici und/oder Phytophtera infestans.

Die nachfolgenden Beispiele dienen der Erläuterung der Erfindung und sind nicht limitierend.

Allgemeine Methoden:

Allgemeine DNA- und Klonierungstechniken, Gelelektrophoresen, Sequenzierung, PCR, Northem-Blots, Expression und Reinigung rekombinanter Proteine, Western-Blots, HPLC- und GC-Analysen sowie die Kultivierung von Mikroorganismen sind gängige Labormethoden und u.a. in Sambrook et al. (Molecular cloning. A laboratory manual (1989) Cold Spring Harbour Laboratory Press) beschrieben.
Die Behandlung von Moos- und Pflanzenmaterial ist ebenfalls gängige Laborpraxis und u.a. in Gelvin et al. (Plant Molecular Biology Manual, 1995, Dovdrecht/Holland, Kluwer Academic Publ.) beschrieben.

Klonierung der Gene kodierend für CYP74-Enzyme via PCR

Für die RACE-PCR standen zwei Lambda ZapII-cDNA-Banken von Physcomitrella patens, eine aus Protonema- und eine aus Gametophytengewebe, zur Verfügung. Als Vektor-Primer diente für die 5'-RACE-PCR der T7-lang Primer (5'-GTA ATA CGA CTC ACT ATA GGG CGA ATT GGG-3'). Die Durchführung erfolgte nach Standardmethoden.

Für die nested RACE-PCR wurde erst eine PCR mit M13rev-Primer (5'-GGA AAC AGC TAT GAC CAT G-3') und einem RACE-Primer durchgeführt Dieser Ansatz diente als Template für eine zweite PCR mit T7-lang Primer und einem (genspezifischen) nested-RACE-Primer ausgewählt aus:

**PP291AOS5R : 5'–TCA CCT CAT CCG ATA CGC TAG TC–3'**

**PP364AOS5R : 5'–GTC GAT GTC GTC TCA ATG TTC C–3'**

**PP364AOS5R2 : 5'–CCA TTC GTG ATT GCC AGA ACT GC–3'**

Zur Klonierung der vollständigen Fragmente wurden diese mit Hilfe des Expand™- PCR-Systems amplifiziert. Neben der *Taq*-Polymerase enthält dieses System eine *Pwo*-Polymerase mit einer Proof-Reading-Aktivität Diese dient dazu, die Lesefehler-rate niedrig zu halten, um möglichst wenig Mutationen in der zu klonierenden DNA zu erhalten. Als Template wurden folgende cDNA-Bänke verwendet: *P. patens*-cDNA-Lambda ZapII aus Protonema und *P. patens*-cDNA-Lambda ZapII aus Gametophyten. Folgende primer wurden eingesetzt:

**PP291/5'SphI**
**5'–AAA GCA TGC ATG GCA GTC CCT TCA TCC AAG C - 3'**

**PP291/3'PstI**
**5'–AAA CTG CAG TCA CTT TTT GAG ATC GGA AAA GAA AAC CTT GGT CGC–3'**

**PP364/5'BamHI**
**5'–GGA TCC CGT ACG GTT GTA GCC AGT CTT GGG–3'**

PP364/3'HindIII
5'-AAG CTT TCA ATC TGA TCG CGG CGT CAG TG-3'

Zur Kontrolle der Klone während der Klonierung von RACE- und vollständigencDNAs wurde eine sogenannte "Kolonie"-PCR mit der Tfl-Polymerase durchgeführt, da hier die Fehlerrate nicht von Bedeutung war. Die Primer wurden beibehalten und das Programm nach Standardvorgaben durchgeführt.

Isolierung von CY*P74*-Enzymen

Aus zwei Lambda ZapII-cDNA Banken von Physcomitrella patens wurden 2 Gene kodierend für CYP74-Enzyme isoliert.

Klon Pp291

Der isolierte Klon Pp291 hatte einen offenen Leserahmen für 322 Aminosäuren. Dieses Protein zeigte 48 % Identität zu einer AOS aus *Parthenium argentatum* (Pan et al.; 1995, *J. Biol. Chem. 270,* 8487-8494). Ein Vergleich seiner Nukleotidsequenz mit anderen bekannten AOS zeigte, dass jedoch noch ca. 500 bis 650 bp am 5'-Ende fehlten. Aus diesem Grund wurde eine 5'-RACE-PCR mit einer Lambda ZapII-cDNA Bank aus Protonema- und einer Lambda Zap II-cDNA-Bank aus Gametophytengewebe durchgeführt. Mit dem RACE-Primer PP291AOS5R (siehe oben) und einer Annealingtemperatur von 60 °C gelang es aus der Protonema-Bank drei Fragmente unterschiedlicher Länge zu amplifizieren. Im Vergleich mit dem im Agarosegel mitgeführten Großenstandard ließen sich die Längen der Fragmente abschätzen. Sie betrugen 600 bp, 700 bp und 800 bp. Die beiden längeren Fragmente wurden in den Vektor pGEM-T kloniert und sequenziert. Die beiden erhaltenen Sequenzen stellten beide eine Verlängerung des 5'-Endes des Ausgangs-Klons dar. In beiden Sequenzen befand sich 15 bp oberhalb vom ersten Start-Codon (ATG) ein Stop-Codon. Für eine Klonierung der vollständigen cDNA wurden, ausgehend von der cDNA-Sequenz aus der RACE-PCR, die Expressionsprimer (PP291/5'SphI und PP291/3'PstI, siehe oben) mit Restriktionsschnittstellen für SphI und PstI abgeleitet. Die erhaltene vollständige cDNA Sequenz ist in SEQ ID No. 1 dargestellt und codiert für ein Protein von 475 Aminosäuren (SEQ ID No. 2).

Klon Pp364

Der zweite cDNA Klon enthielt einen offenen Leserahmen, der für ein Protein von 489 Aminosäuren codierte. Dieses zeigte auf Aminosäureebene 42 % Identität zur AOS aus *Arabidopsis thaliana* (Laudert et al.; 1996, *Plant Mol. Biol. 31,* 323-335) und 41 % Identität zur AOS aus *L. usitatissimum.* Dieser Klon enthielt trotz seiner Länge kein Start-Codon. Die Expressionsprimer PP364/5'BamHI und PP364/3'HindIII mit den Restriktionsschnittstellen für BamHI und HindIII wurden aus der bekannten Klon-Sequenz abgeleitet. Durch RACE-PCR und inverted PCR wurde die vollständige cDNA-Sequenz erhalten. Die vollständige cDNA-Sequenz ist in SEQ ID No. 3 dargestellt und kodiert für ein Protein von 532 Aminosäuren (SEQ ID No. 4).

Expression und Reinigung rekombinanter Proteine

Für die Expression mussten die isolierten cDNAs in einen Expressionsvektor ligiert werden. Hier wurde der Vektor pQE30 (Qiagen, Hilden) verwendet, um die Proteine mit N-terminalen $His_6$-Tag zu exprimieren. Die Ligation zwischen vorgeschnittenen pQE30 und Donor-DNA (im Verhältnis ca. 3:1) erfolgte mit T4-DNA Ligase.
Die Expression der rekombinanten Proteine erfolgte im *E. coli*-Stamm SG13009 (Gottesmann et al.; 1981, *J. Bacteriol. 148*, 265-73). Die Expressionsklone wurden zunächst bis zu einer $OD_{600}$ von 0,6 - 0,8 in LB-Medium (mit Carbenicillin und Kanamycin) bei 37 °C inkubiert Nach der Induktion mit IPTG (Endkonzentration: 1 mM) wurden die Bakterien für weitere 2-3 Tage bei 10 °C kultiviert.
Die Reinigung erfolgte soweit wie möglich auf Eis, die Zentrifugationsschritte erfolgten bei 4 °C. Die Zellen wurden durch eine Zentrifugation bei 4000 x g 15 min sedimentiert.
Dieses Zellsediment wurde vollständig in 50 mM Na-Phosphat, pH 8, resuspendiert und mit Ultraschall (Sonopuls GM 70, Bandelin, Berlin) für 5 x 1 min bei 50 % Leistung und 50 % Impuls aufgeschlossen. Die Zelltrümmer wurden 15 min bei 4000 x g abzentrifugiert und aus dem Überstand wurden durch eine weitere Zentrifugation (1 h bei 100000 x g) die Zellmembranen sedimentiert.
Die Reinigung der rekombinant hergestellten Proteine erfolgte für beide Klone Pp291 und Pp364 über eine Affinitätschromatographie. Anschließend wurde mit einem pH-Gradienten (Fig. 1A) und alternativ mit einem Imidazol-Gradienten (Fig. 1B) eluiert. Von allen Wasch- und Elutionsschritten wurden gleiche Volumina (1 ml) gefällt und auf das Gel aufgetragen.

Wie in Fig. 1A zu sehen ist, wurde sauberes Pp291-Protein bei einem pH von 4 von der Säule eluiert. Beim Imidazolgradient eluierte das Protein ab einer Imidazol-Konzentration von 100 mM relativ sauber. Beim Vergleich beider Methoden konnte kein Unterschied festgestellt werden. Aus diesem Grund wurde im folgenden das Protein nur noch mit dem pH-Gradient gereinigt.

Aktivitätstests

Da Sequenzvergleiche keine Rückschlüsse auf die katalytische Aktivität zulassen, wurden die bei der Katalyse der Enzyme entstehenden Produkte mittels einem photometrischen Aktivitätstest sowie HPLC- und GC-Analysen analysiert, die nach Standardmethoden durchgeführt wurden.

Beim photometrischen Aktivitätstest wird die Abnahme der Extinktion bei 234 nm gemessen. Sie beruht auf der Zerstörung des konjugierten DienSystems der Hydroperoxide beim Umsatz mit *Cyp74*-Enzymen. Dazu wurde in Phosphatpuffer (pH je nach pH-Optimum des Enzyms) das entsprechende Substrat gelöst (zum reinen Aktivitätstest in der Regel eine Endkonzentration von 20 μM 13-HPOTE bzw. 9-HPODE) und die Reaktion durch Zugabe des Enzyms gestartet Die Substratkonzentration wurde photometrisch über die Extinktion bei 234 nm verfolgt. Der molare Extinktionskoeffizient ($\lambda_{234}$nm) ist gleich 25000 $M^{-1}cm^{-1}$.

Es wurden sowohl von dem Enzym kodiert durch den Klon Pp291 als auch von dem Enzym kodiert von dem Klon Pp364 beide Substrate (13-HPOTE und 9-HPODE) umgesetzt. Eine graphische Darstellung findet sich in Fig. 2.

Bei der Umsetzung von Hydroperoxiden mit DES entstehen Divinylether, die im sauren zu Aldehyden hydrolysieren. Diese sind leicht flüchtig. Um diese entstehenden Aldehyde zu identifizieren, besteht die Möglichkeit sie mit DNPH zu derivatisieren (Kohlmann et al.; 1999, *Eur. J. Biochem. 260,* 885-895). Die so gebildeten Hydrazone sind nicht mehr flüchtig und können über ihre Retentionszeit mit Hilfe einer HPLC-Analyse identifiziert werden. Wie Fig. 3 zeigt, können auch Divinylether bei der Behandlung mit dem Derivatisierungsreagenz in Aldehyde zerfallen, die dann als DNPH-Derivate nachgewiesen werden können.

Mit dieser Methode konnte gezeigt werden, dass in dem Reaktionsansatz des Enzyms kodiert durch den Klon Pp364 mit den Substraten 13-HPOTE und 9-HPODE Aldehyde entstehen (Fig. 4). Diese wurden als (2*E*)-Hexenal und (2E)-Nonenal anhand ihrer Retentionszeiten identifiziert. Sie zeigten auch ein typisches UV-Spektrum für Aldehyd-DNPH-Derivate. Bei Pp291 wurden keine Aldehyde nachgewiesen (Daten nicht gezeigt).

Bei der HPLC-Analyse der nicht flüchtigen Verbindungen setzt man Hydroperoxide um, die $1\text{-}^{14}C$ markiert sind. Es entstehen markierte Produkte, die mit Hilfe eines Radiodetektors anstelle eines UV-Detektors sichtbar gemacht werden können. So kann man auch Substanzen, die kein typisches UV-Maximum besitzen über ihre Retentionszeit identifizieren. Ein weiterer Vorteil ist die höhere Sensitivität dieser Detektion gegenüber der UV-Detektion. Setzt man $[1\text{-}^{14}C]$-13-HPOTE als Substrat in der Enzymreaktion ein, so zeigt sich in dem in Fig. 5 dargestellten Chromatogramm für Pp291 das gleiche Elutionsprofil wie die 9/13-AOS1 aus Gerste (Maucher et al.; 2000, *Plant J. 21*, 199-213), die als Referenz verwendet wurde. Bei anderen AOSs wurde bereits gezeigt, dass das Hauptprodukt der Reaktion in Abwesenheit von Allenoxid-Cyclase das α-Ketol ist.

Das Chromatogramm der Expression und dem anschließenden Umsatz von Pp364 zeigte ein Signal bei ca. 30 min (Fig. 6). Dieses wurde weder bei der 9/13-HPL der Gurke noch bei der 9/13-AOS1 der Gerste gefunden. Pp364 zeigte zusätzlich noch Signale, mit der gleichen Retentionszeit wie die Produkte der 9/13-HPL der Gurke.

Da bei der Radio-HPLC dem Säuleneluat Flüssigszintillator zugesetzt wird, wurde der gleiche HPLC-Lauf mit nicht markierten Substraten durchgeführt, um die Produkte aufzufangen und weiter zu analysieren. Da α-Ketole kein typisches UV-Maximum besitzen erfolgte hier die Detektion bei 210 nm (Gardner, 1997, *Advances in Lipid Methodology - four* (Christie, W. W., ed) pp. 1-43, The Oily Press, Dundee).

Bei Pp291 wurde eine Substanz mit der gleichen Retentionszeit wie der Standard für das α-Ketol, das aus 13-HPOTE entsteht, aufgefangen, derivatisiert und mit Hilfe des GC/MS untersucht Dabei zeigte sich das in (Fig. 7) dargestellte Chromatogramm. Das Massenspektrum ist identisch mit dem des entsprechenden α-Ketol-Standards.

Bestimmung von Enzymcharakteristika

pH-Optimum

Das pH-Optimum wurde für den Klon Pp291 am Photometer bestimmt Dazu wurde 13-HPOTE als Substrat genutzt (ca. 25 μM in 50 mM Na-Phosphat des entsprechenden pH-Wertes). Die größte Aktivität zeigt das Enzym in einem pH-Bereich von 5,0 bis 6,0. Dies ist in Fig. 8 dargestellt

Enzymkinetische Parameter

Pp291 wurde hinsichtlich seiner Substratspezifität vor allem gegenüber Hydroperoxiden der Arachidonsäure (HPETE) untersucht, da sie die dominierende Fettsäure dieses Organismus darstellt (Girke et al.; 1998, *Plant J. 15,* 39-48). Dabei zeigte sich das in Fig. 9 dargestellte Verhältnis der Umsetzungsgeschwindigkeiten. Demnach wird 8-HPETE von den hier untersuchten Hydroperoxiden am schnellsten umgesetzt. Danach folgen 13-HPOTE, 9-HPODE und 11-HPETE im Verhältnis 8-HPETE : 13-HPOTE : 9-HPODE : 11-HPETE,100 : 70 : 60 : 57.

<u>Überexpression zur Erzeugung einer erhöhten Pathogenresistenz</u>

Zur Klonierung in binäre Vektoren werden die cDNAs von Pp291 und Pp364 mit BamHI und NotI restringiert und in einen mit BamHI und NotI geschnittenen pCRScript-Vektor überführt. Aus diesem Vektor werden sie anschliessend mit SalI herausgeschnitten und in einen entsprechend mit SalI geschnittenen pBinAR Vektor ligiert. Durch Kontrollschnitte mit BamHI und Sequenzierung werden die Klone ermittelt, in denen die Gene in sense-Orientierung vorliegen.

Transformation in Arabidopsis thaliana:

Unter Verwendung dieser pBinAR Vektoren enthaltend je eine Pp291 und Pp364 cDNA in sense-Orientierung wird Agrobacterium tumefaciens (C58C1 pMP90) transformiert. Anschließend werden Wildtyp Arabidopsis thaliana-Pflanzen (cv. Columbia) mit dem jeweiligen transformierten Agrobacterium tumefaciens Stamm auf Grundlage einer modifizierten Methode der Vakuum Infiltrationsmethode nach Clough und Bent (Clough, S. and Bent A., Plant J 1998, 16(6):735-43) und nach Bechtold, et al. (Bechtold, N. et al., CRAcad Sci Paris 1993, 1144(2):204-212) transformiert.

Samen der Primärtransformanden werden auf Grundlage der Kanamycin-Resistenz gescreent, indem Saatgut auf Kanamycin-haltigen MS-Platten (MS-Medium (Sigma) mit 40mg/l Kanamycin, 10mg/l Benomyl und 100mg/l Timentin) ausgelegt wird. Kanamycin-resistente Keimlinge werden nach 2 Wochen in Erde transferiert und als vollentwickelte Pflanzen zur phänotypischen und molekularen Analyse verwendet.

Aus den transgenen Pflanzen, die mit Pp291 und Pp364 in sense-Orientierung transformiert worden sind, werden diejenigen ausgewählt, in denen es zu keinem Kosuppressionseffekt gekommen ist. Dazu wird gesamt-RNA aus den Pflanzen isoliert und mittels Real-Time PCR (Perkin-Elmer) das Vorliegen des Messengers nachgewiesen. Die so identifizierten transgenen Pflanzen wurden mit verschiedenen Pathogenen infiziert. Dazu zählten Blumeria graminis f.sp. hordei und f.sp. tritici, Pythium spec., Albugo spec., Rhizoctonia solani, Peronospora parasitica, Erysiphe cruciferarum, E. cichoreacearum, Alternaria brassicicola, Botrytis cinerea, Sclerotium rolfsii, Sclerotinia sclerotium, Fusarium oxysporum, F. culmorum, F. graminearum, F. nivale oder Pseudomonas syringae.

Das Ergebnis, d.h. der makroskopische Resistenzgrad der Pflanze gegenüber dem pathogenen Pilz bzw. Bakterium, wurde u.a. unter Zuhilfenahme von Fluoreszenz- und Lichtmikroskopie analysiert. Dabei zeigte sich, dass die Pp291- und Pp364-transgenen *Arabidopsis*-Pflanzen eine erhöhte Resistenz gegen die erwähnten Pathogene aufwiesen im Vergleich zum Wildtyp.

Transformation in Gerste:

Gerste cv Pallas Blattsegmente wurden mit einer cDNA von Pp291 bzw. Pp364, die in dem GFP-Expressionsvektor (Green Fluorescence Protein) vorlagen, transformiert. Anschließend wurden die Blätter mit dem pathogenen Pilz Blumeria graminis f.sp. hordei (Echter Gerstenmehltau) inokuliert und das Ergebnis nach 48 h mittels Licht- und Fluoreszenzmikroskopie analysiert. Die Penetration in GFP-exprimierenden Zellen wurde mittels Detektion von Haustorien in lebenden Zellen und durch Bewertung der Pilzentwicklung in eben diesen Zellen beurteilt. In allen sechs Experimenten führte die Bombardierung von Gerste cv Pallas mit Pp291- bzw. Pp364-cDNA zu einer verminderten Anzahl von erfolgreich durch Blumeria graminis f.sp. hordei (Echter Gerstenmehltau) penetrierten Zellen im Vergleich zu Zellen die mit einer fremden Kontroll-cDNA (humaner Thyroidhormonrezeptor dsRNA, TR) bombardiert wurden. Der resistenzinduzierende Effekt der Pp291- bzw. Pp364-cDNA bedingte eine durchschnittliche Verminderung der Penetrationseffizienz durch Blumeria graminis f.sp. hordei um 44%.

Zur transienten Transformation von Gerste wurde ein Verfahren eingesetzt das bereits für die biloistische Einführung von cDNA in epidermale Zellen von Gerstenblättem beschrieben wurde (Schweizer P et al. (1999) Mol Plant Microbe Interact 12:647-54; Schweizer P et al. (2000) Plant J 2000 24: 895-903). Wolframpartikel mit einem Durchmesser von 1,1 mm (Partikeldichte 25 mg/ml) wurden mit cDNA zusammen mit Plasmid-DNA des Vektors pGFP (GFP unter Kontrolle des CaMV 35S Promotors) als Transformationsmarker beschichtet. Dazu wurden pro Schuss die nachfolgender Mengen an cDNA bzw. Reporterplasmid zur Beschichtung verwendet: 1 mg pGFP und 2 mg cDNA. Zur Microcarrier-Präparation wurden 55 mg Wolframpartikel (M 17, Durchmesser 1,1 mm; Bio-Rad, München) zweimal mit 1 ml autoklaviertem destilliertem Wasser und einmal mit 1 ml absolutem Ethanol gewaschen, getrocknet und in 1 ml 50 %igem Glycerin aufgenommen (ca. 50 mg/ml Stammlösung). Die Lösung wurde mit 50 %igern Glycerin auf 25 mg/ml verdünnt, vor Gebrauch gut gemischt und im Ultraschallbad suspendiert. Zur Microcarrier-Beschichtung wurden pro Schuss 1 mg Plasmid, 2 mg cDNA (1 mL), 12,5 ml Wolframpartikel-Suspension (25 mg/ml), 12,5 ml 1 M Ca(NO3)2-Lösung (pH 10) tropfenweise unter ständigem Mischen zusammengegeben, 10 min bei RT stehengelassen, kurz zentrifugiert und 20 ml vom Überstand abgenommen. Der Rest mit den Wolframpartikeln wird resuspendiert (Ultraschallbad) und ins Experiment eingesetzt

Es wurden ca. 4 cm lange Segmente von Gerstenprimärblättern verwendet Die Gewebe wurden auf 0,5 % Phytagar (GibcoBRLt Life Technologiest, Karlsruhe) mit 20 mg/ml Benzimidazol in Petrischalen (6,5 cm Durchmesser) gelegt und direkt vor dem Partikelbeschuss an den Rändern mit einer Schablone mit einer rechteckigen Aussparung von 2,2 cm x 2,3 cm abgedeckt Die Schalen wurden nacheinander auf den Boden der Vakuumkammer (Schweizer P et al. (1999) Mol Plant Microbe Interact 12:647-54) gestellt über dem ein Nylonnetz (Maschenweite 0,2 mm, Millipore, Eschbom) als Diffusor auf einer Lochplatte eingeschoben war (5 cm über dem Boden, 11 cm unterhalb des Macrocarriers, s.u.), um Partikelklumpen zu zerstreuen und den Partikelstrom abzubremsen. Der oben an der Kammer angebrachte Macrocarrier (Plastik-Sterilfilterhalter, 13 mm, Gelman Sciences, Swinney, UK) wurde je Schuss mit 5,8 ml DNAbeschichteten Wolframpartikeln (Microcarrier, s.u.) beladen. Mit einer Membranvakuumpumpe (Vacuubrand, Wertheim) wurde der Druck um 0,9 bar in der Kammer reduziert und die Wolframpartikel mit 9 bar Heliumgasdruck auf die Oberfläche des Pftanzengewebes geschossen. Sofort danach wurde die Kammer belüftet.

Zur Markierung transformierter Zellen wurden die Blätter mit dem Plasmid (pGFP; Vektor auf pUC18-Basis, CaMV 35S-Promoter/Terminator-Kassette mit insertiertem GFP-Gen; Schweizer P et al. (1999) Mol Plant Microbe Interact 12:647-54; zur Verfügung gestellt von Dr. P. Schweizer Schweizer P, Institut für Pflanzengenefik IPK, Gatersleben, Deutschland) beschossen. Vor dem Schießen eines anderen Plasmids wurde der Macrocarrier jeweils gründlich mit Wasser gereinigt Nach vierstündiger Inkubation nach dem Beschuss bei leicht geöffneten Petrischalen, RT und Tageslicht wurden die Blätter mit 100 Konidien/mm des Echten Gerstenmehltaupilzes (Rasse A6) inokuliert und für weitere 40 bis 48 h unter gleichen Bedingungen inkubiert.

Blattsegmente wurden mit den beschichteten Partikeln unter Verwendung einer "particle inflow gun" bombardiert. Pro Schuss wurden 312 mg Wolframpartikel appliziert. 4 h nach der Bombardierung wurde Inokulation mit Blumeria graminis Mehltau (Rasse A6) inokuliert und nach weiteren 40 h bezüglich der Infektionsanzeichen ausgewertet Das Ergebnis (z.B. die Penetrationseffizienz, definiert als prozentualer Anteil angegriffener Zellen, die ein reifes Haustorium und eine Sekundärhyphe ("secondary elongating hyphae") ausbilden; Berechnung siehe weiter unten) wurde mittels Fluoreszens- und Lichtmikroskopie analysiert. Eine Inokulation mit 100 Conidia/mm2 ergibt eine Angriffsfrequenz von ca. 50 % der transformierten Zellen. Für jedes einzelne Experiment wurde eine minimale Anzahl von 100 Interaktionsstellen ausgewertet Transformierte (GFP exprimierende) Zellen wurden unter Anregung mit blauem Licht identifiziert Drei verschiedene Katagorien von transformierten Zellen konnten unterschieden werden:

1. Penetrierte Zellen, die ein leicht erkennbares Haustorium beinhalten. Eine Zelle mit mehr als einem Haustorium wurde als eine Zelle gewertet.
2. Zellen, die durch ein Pilz-Appressorium zwar angegriffen wurden, aber kein Haustorium beinhalten. Eine Zelle die mehrfach von Blumeria graminis ssp. hordeum_angegriffen wurden, aber kein Haustorium enthält, wurde als eine Zelle gewertet
3. Zellen die nicht durch Blumeria graminis ssp. hordeum angegriffen sind.

Stomatazellen und Stomatanebenzellen wurden von der Bewertung ausgeschlossen. Oberflächenstrukturen von Blumeria graminis ssp. hordeum wurden mittels Lichtmikroskopie und Fluoreszenzfärbung des Pilzes mit 0.1 % Calcofluor(w/v in Wasser) für 30 sec analysiert. Die Entwicklung des Pilzes kann leicht durch Fluoreszenzmikroskopie nach Anfärbung mit Calcofluor evaliert werden. In Pp291- bzw. Pp364-dsRNA transformierten Zellen entwickelt der Pilz zwar ein primäres und ein appressoriales "Germ-Tube" aber kein Haustorium. Haustoriumausbildung ist eine Vorbedingung für die Bildung einer Sekundärhyphe.

Die relative Penetrationseffizien (RPE) erreichnet sich als Differenz aus der Penetrationseffizien bei transformierten Zellen (Transformation mit Pp291- bzw. Pp364-cDNA) und der Penetrationseffizienz bei untransformierten Zellen (hier: durchschnittliche Penetrationseffizienz 57%). Die prozentuale RPE (%-RPE) errechnet sich aus der RPE minus 1 und multipliziert mit 100.

RPE = [PE bei transformierten Zellen]
[PE bei untransformierten Zellen]

$$\%\text{-RPE} = 100 \times (\text{RPE}-1)$$

Der %-RPE-Wert (Abweichung von der durchschnittlichen Penetrationseffizienz der Kontrolle) dient der Bestimmung des Suszeptibilität von Zellen, die mit pp291- bzw. Pp364-cDNA transfiziert sind.

Bei der Kontroll-cDNA wurde bei fünf unabhängigen Versuchen kein Unterschied zwischen der Transfektion mit der Kontroll-cDNA und Wasser bezüglich der Penetrationseffizienz von Blumeria graminis ssp. hordeum beobachtet. Um einen Einfluss der cDNA auf die Transformationsrate oder Überlebensrate der angegriffenen Zellen auszuschließen, wurde die Anzahl der GFP-exprimierenden Zellen zwischen Kontroll- und Pp291- bzw. Pp364-cDNA Experimenten verglichen. Die Pp291- bzw. Pp364-cDNA hatten keinen Einfluß auf die Gesamtanzahl- oder die Anzahl der angegriffenen GFP-exprimierenden Zellen.

Die Transfektionsrate mit Pp291 und Pp364 führte zu einer drastischen Abnahme der Penetrationsfrequenz von Blumeria graminis f.sp. hordei (durchschnittlicher %-RPE-Wert = -30%).

Figurenbeschreibung:

Figur 1: Affinitätsaufreinigung des Klons Pp291 (**A**) Elution mit pH-Gradient M = Marker; 1 = Leervektor pQE30 (nicht induziert); 2 = pQE30 (mit IPTG induziert); 3 = nicht induzierter Pp291; 4 = induzierter Pp291, 5 = Überstand nach 1. Ultrazentrifugation; 6 = Überstand nach 2. Ultrazentrifugation; 7 = Durchfluß; 8, 9,10 = Waschschritte pH 8, pH 7, pH 6; 11,12 = Elutionsschritte pH 5, pH 4 ; (**B**) Elution mit Imidazolgradient: M = Marker; 1 bis 11 steigende Imidazolkonzentration (mM): 0, 20, 40, 60, 80,100, 125, 150, 200, 250, 300.

Figur 2: Photometrischer Aktivitätstest: Dargestellt ist die Abnahme der Absorption bei 234 nm einer Hydroperoxid-Lösung nach Zusatz eines Zelllysats des Klons Pp291 (C), einer 1:50 Verdünnung dieses Zelllysats (B) bzw. des Leervektors pQE30 (A).

Figur 3: Elutionsprofil der HPLC-Analyse von Hydrazonen als Derivate flüchtiger Aldehyde, die bei der Umsetzung von Hydroperoxiden mit Divinylether-Synthase entstehen: Nachweis von (2E)-Nonenal nach Behandlung von Colnelsäure (einem Divinylether) mit dem Derivatisierungsreagenz für Aldehyde.

Figur 4: Elutionsprofil der HPLC-Analyse von Katalyseprodukten (Aldehyden) des Enzyms DES kodiert durch den Klon Pp364 mit den Substraten 13-HPOTE und 9-HPODE. Diese wurden als (2E)-Hexenal und (2E)-Nonenal anhand ihrer Retentionszeiten identifiziert.

Figur 5: Elutionsprofil der HPLC-Analyse nicht-flüchtiger Produkte der Umsetzung von Hydroperoxiden durch das Enzym AOS kodiert durch den Klon Pp291.

Figur 6: Elutionsprofil der HPLC-Analyse nicht-flüchtiger Produkte der Umsetzung von Hydroperoxiden durch das Enzym DES kodiert durch den Klon Pp364;
a = Zellysat; b = gereinigtes Enzym.

Figur 7: GC/MS-Chromatogramm des Produkts des durch Katalyse des CYP74-Enzyms kodiert durch den Klon Pp291 entstanden ist. Das Signal bei 18,2 min hat das im Fenster dargestellte Massenspektrum.

Figur 8: Relative Enzymaktivität des Enzyms kodiert durch den Klon Pp291 in Abhängigkeit vom pH-Wert.

Figur 9: Relative Umsetzungsgeschwindigkeiten verschiedender Substrate durch das CYP74-Enzym kodiert durch den Klon Pp291 (Substratspezifität); Substratkonzentration jeweils 20 μM; die Bestimmung erfolgte am Photometer; n. m. = nicht messbar

**Patentansprüche**

1. Allenoxid-Synthase mit einer Aminosäuresequenz gemäß SEQ ID No. 2.

2. Divinylether-Synthase mit einer Aminosäuresequenz gemäß SEQ ID No. 4.

3. Enzyme gemäß einem der Ansprüche 1 oder 2, **dadurch gekennzeichnet, daß** sie sowohl 9-HPOD/TE als auch 13-HPOD/TE als Substrat umsetzen.

4. Isolierte Nukleotidsequenz kodierend für eine Allenoxid-Synthase gemäß Anspruch 1 beteiligt an der Biosynthese von mehrfach ungesättigten Fettsäuren ausgewählt aus

   a) einer Nukleotidsequenz gemäß SEQ ID No. 1,
   b) einer Nukleotidsequenz, die wenigstens 70% Identität mit der in SEQ ID No. 1 gezeigten Nukleotidsequenz aufweist,
   c) einer zu a) oder b) komplementären Nukleotidsequenz.

5. Isolierte Nukleotidsequenz kodierend für eine Divinylether-Synthase gemäß Anspruch 2 beteiligt an der Biosynthese von mehrfach ungesättigten Fettsäuren ausgewählt aus

   a) einer Nukleotidsequenz gemäß SEQ ID No. 3,
   b) einer Nukleotidsequenz, die wenigstens 70% Identität mit einer der in SEQ ID No. 3 gezeigten Nukleotidsequenz aufweist,
   c) einer zu a) oder b) komplementären Nukleotidsequenz.

6. Isolierte Nukleotidsequenz gemäß einem der Ansprüche 4 oder 5, **dadurch gekennzeichnet, daß** sie aus Moos oder höheren Pflanzen stammt.

7. Isolierte Nukleotidsequenz gemäß einem der Ansprüche 4-6, **dadurch gekennzeichnet, daß** sie aus Physcomitrella patens stammt.

8. Genkonstrukt enthaltend wenigstens eine Nukleotidsequenz gemäß einem der Ansprüche 4-7 sowie operativ damit verknüpfte regulatorische Nukleotidsequenzen.

9. Vektor enthaltend wenigstens eine isolierte Nukleotidsequenz gemäß einem der Ansprüche 4-7 und/oder ein Genkonstrukt gemäß Anspruch 8 sowie zusätzliche Nukleotidsequenzen zur Selektion und/oder Replikation in einer Wirtszelle und/oder zur Intregration in das Genom einer Wirtszelle.

10. Transgene Pflanzenzelle, ganze Pflanze und/oder deren Nachkommen, enthaltend in replizierbarer Form wenigstens eine isolierte Nukleotidsequenz gemäß einem der Ansprüche 4-7 und/oder ein Genkonstrukt gemäß Anspruch 8 und/oder einen Vektor gemäß Anspruch 9, **dadurch gekennzeichnet, daß** sie eine verstärkte Expression der Nukleotidsequenz kodierend für eine Allenoxid-Synthase und/oder der Nukleotidsequenz kodierend für eine Divinylether-Synthase im Vergleich zu der endogen vorliegenden Genexpression aufweist, die zu einer gesteigerten Resistenz von Pflanzen gegenüber Krankheitserregern führt.

11. Transgene Pflanzenzelle, ganze Pflanze und/oder deren Nachkommen gemäß Anspruch 10, **dadurch gekennzeichnet, daß** wenigstens eine Nukleotidsequenz gemäß einem der Ansprüche 4-7 und/oder ein Genkonstrukt gemäß Anspruch 8 und/oder ein Vektor gemäß Anspruch 9 extrachromosomal vorliegt und/oder stabil in das pflanzliche Genom integriert ist.

12. Transgene Pflanzenzelle, ganze Pflanze und/oder deren Nachkommen gemäß einem der Ansprüche 10 oder 11, welche eine Allenoxid-Synthase gemäß Anspruch 1 und/oder eine Divinylether-Synthase gemäß Anspruch 2 mit einer erhöhten spezifischen Aktivität, im Vergleich zu der entsprechenden endogenen spezifischen Enzymaktivität in einer Pflanzenzelle, aufweist.

13. Transgene Pflanzenzelle, ganze Pflanze und/oder deren Nachkommen gemäß einem der Ansprüche 10-12, **dadurch gekennzeichnet, daß** sie eine Nützpflanze oder deren Zellen, bevorzugt der Familie der Solanaceae oder Getreide ist, besonders bevorzugt Kartoffel, Gerste oder Weizen ist.

14. Verfahren zur Steigerung der Resistenz von Pflanzen gegenüber Krankheitserregern, **dadurch gekennzeichnet, daß** eine Nukleotidsequenz gemäß einem der Ansprüche 4-7 einzeln oder in Kombination und/oder ein Genkonstrukt gemäß Anspruch 8 und/oder ein Vektor gemäß Anspruch 9 in replizierbarer Form in Pflanzenzellen übertragen wird und aus den so transformierten Pflanzenzellen ganze Pflanzen regeneriert werden.

15. Verfahren gemäß Anspruch 14, **dadurch gekennzeichnet, daß** als pflanzliche Zellen solche von Nutzpflanzen, bevorzugt der Familie der Solanaceae oder von Getreide, besonders bevorzugt Kartoffel, Gerste oder Weizen eingesetzt werden.

16. Verwendung wenigstens einer Nukleotidsequenz gemäß einem der Ansprüche 4-7 zur Steigerung der Resistenz von Pflanzenzellen, ganzen Pflanzen und/oder deren Nachkommen gegenüber Krankheitserregern.

17. Verwendung von wenigstens einem Enzym gemäß einem der Ansprüche 1-6 zur Steigerung der Resistenz von Pflanzenzellen, ganzen Pflanzen und/oder deren Nachkommen gegenüber Krankheitserregern.

18. Verwendung gemäß Anspruch 17, wobei eine erhöhte spezifische Aktivtät wenigstens eines Enzyms gegenüber der entsprechenden endogenen spezifischen Enzym-Aktivität eine Steigerung der Resistenz der Pflanzenzellen oder Pflanzen gegenüber Krankheitserregern um 20-90%, bevorzugt um 30-80% und besonders bevorzugt um 40-70% bewirkt.

19. Verwendung gemäß einem der Ansprüche 17 oder 18, wobei wenigstens ein Enzym eine erhöhte Resistenz gegenüber Mehltaupilzen, bevorzugt Blumeria graminis f. sp. hordei oder f. sp. tritici und/oder Phytophtora infestans bewirkt.

**Claims**

1. An allene oxide synthase with an amino acid sequence as shown in SEQ ID No. 2.

2. A divinyl ether synthase with an amino acid sequence as shown in SEQ ID No. 4.

3. Enzymes according to claim 1 or 2, which convert both 9-HPOD/TE and 13-HPOD/TE as substrate.

4. An isolated nucleotide sequence encoding an allene oxide synthase according to claim 1 which is involved in the biosynthesis of polyunsaturated fatty acids selected from among

   a) a nucleotide sequence as shown in SEQ ID No. 1,
   b) a nucleotide sequence with at least 70% identity with the nucleotide sequence shown in SEQ ID No. 1,
   c) a nucleotide sequence which is complementary to a) or b).

5. An isolated nucleotide sequence encoding a divinyl ether synthase according to claim 2 which is involved in the biosynthesis of polyunsaturated fatty acids selected from among

   a) a nucleotide sequence as shown in SEQ ID No. 3,
   b) a nucleotide sequence with at least 70% identity with a the nucleotide sequence shown in SEQ ID No. 3,
   c) a nucleotide sequence which is complementary to

   a) or b).

6. The isolated nucleotide sequence according to claim 4 or 5, which originates from moss or higher plants.

7. The isolated nucleotide sequence according to any of claims 4-6, which originates from Physcomitrella patens.

8. A gene construct comprising at least one nucleotide sequence according to any of claims 4-7, and regulatory nucleotide sequences operatively linked thereto.

9. A vector comprising at least one isolated nucleotide sequence according to any of claims 4-7, and/or a gene construct according to claim 8 and additional nucleotide sequences for selection and/or replication in a host cell and/or for integration into the genome of a host cell.

10. A transgenic plant cell, intact plant and/or their progeny, comprising, in replicable form, at least one isolated nucleotide sequence according to any of claims 4-7, and/or a gene construct according to claim 8 and/or a vector according to claim 9, which shows increased expression of the nucleotide sequence encoding an allene oxide synthase and/or of the nucleotide sequence encoding a divinyl ether synthase in comparison with the endogenous gene expression, which brings about an increased resistance of plants to pathogens.

11. The transgenic plant cell, intact plant and/or their progeny according to claim 10, wherein at least one nucleotide sequence according to any of claims 4-7, and/or a gene construct according to claim 8 and/or a vector according to claim 9 is present in extrachromosomal form and/or integrated stably into the plant genome.

12. The transgenic plant cell, intact plant and/or their progeny according to claim 10 or 11 with an allene oxide synthase according to claim 1, and/or a divinyl ether synthase according to claim 2, with an increased specific activity in comparison with the corresponding endogenous specific enzyme activity in a plant cell.

13. The transgenic plant cell, intact plant and/or their progeny according to any of claims 10-12, which is a useful plant or cells thereof, preferably from the Solanaceae family or the cereal family, especially preferably potato, barley or wheat.

14. A method for increasing the resistance of plants to pathogens, which comprises transferring, into plant cells, a nucleotide sequence according to any of claims 4-7, either individually or in combination, and/or a gene construct according to claim 8 and/or a vector according to claim 9 in replicable form and regenerating intact plants from the plant cells transformed thus.

15. The method according to claim 14, wherein the plant cells employed are cells from useful plants, preferably from the Solanaceae family or from cereals, especially preferably potato, barley or wheat.

16. The use of at least one nucleotide sequence according to any of claims 4-7 for increasing the resistance of plant cells, intact plants and/or their progeny to pathogens.

17. The use of at least one enzyme according to any of claims 1-6 for increasing the resistance of plant cells, intact plants and/or their progeny to pathogens.

18. The use according to claim 17, wherein an increased specific activity of at least one enzyme brings about an increase in the resistance of the plant cells or plants to pathogens by 20-90%, preferably by 30-80%, especially preferably by 40-70%, in comparison with the corresponding endogenous specific enzyme activity.

19. The use according to claim 17 or 18, wherein at least one enzyme brings about an increased resistance to mildews, preferably Blumeria graminis f.sp. hordei or f.sp. tritici and/or Phytophtora infestans.

**Revendications**

1. Allénoxyde synthase qui présente la séquence d'acides aminés selon SEQ ID No. 2.

2. Divinyléther synthase qui présente la séquence d'acides aminés selon SEQ ID No. 4.

3. Enzymes selon l'une des revendications 1 ou 2, **caractérisées en ce qu'**elles transforment tant la 9-HPOD/TE que la 13-HPOD/TE comme substrats.

4. Séquence isolée de nucléotides qui code une allénoxyde synthase selon la revendication 1 qui participe à la biosynthèse d'acides gras polyinsaturés et qui est sélectionnée parmi :

   a) la séquence de nucléotides selon SEQ ID No. 1,
   b) une séquence de nucléotides qui présente au moins 70 % d'identité avec la séquence de nucléotides présentée dans SEQ ID No. 1 et
   c) une séquence de nucléotides complémentaire à a) ou b).

5. Séquence isolée de nucléotides qui code pour une divinyléther synthase selon la revendication 2, qui participe à la biosynthèse d'acides gras polyinsaturés et qui est sélectionnée parmi :

   a) la séquence de nucléotides selon SEQ ID No. 3,
   b) une séquence de nucléotides qui présente au moins 70 % d'identité avec la séquence de nucléotides présentée dans SEQ ID No. 3 et
   c) une séquence de nucléotides complémentaire à a) ou b).

6. Séquence isolée de nucléotides selon l'une des revendications 4 ou 5, **caractérisée en ce qu'**elle est extraite de mousses ou de plantes supérieures.

7. Séquence isolée de nucléotides selon l'une des revendications 4 à 6, **caractérisée en ce qu'**elle est extraite de Physcomitrella patens.

8. Structure génétique qui comprend au moins une séquence de nucléotides selon l'une des revendications 4 à 7 et des séquences de nucléotides de régulation qui y sont efficacement combinées.

9. Vecteur qui contient au moins une séquence isolée de nucléotides selon l'une des revendications 4 à 7 et/ou une structure génétique selon la revendication 8 ainsi que des séquences de nucléotides supplémentaires destinées à être sélectionnées et/ou répliquées dans une cellule hôte et/ou à être intégrées dans le génome d'une cellule hôte.

10. Cellule végétale transgénique, plante complète et/ou leurs descendants qui comprennent sous forme réplicable au moins une séquence isolée de nucléotides selon l'une des revendications 4 à 7 et/ou une structure génétique selon la revendication 8 et/ou un vecteur selon la revendication 9, **caractérisés en ce que** la séquence de nucléotides qui code pour l'allénoxyde synthase et/ou la séquence de nucléotides qui code pour la divinyléther synthase sont plus fortement exprimées qu'avec le gène endogène présent, ce qui renforce la résistance des plantes contre les agents pathogènes.

**11.** Cellule végétale transgénique, plante complète et/ou leurs descendants selon la revendication 10, **caractérisés en ce qu'**au moins une séquence de nucléotides selon l'une des revendications 4 à 7, et/ou une structure génétique selon la revendication 8 et/ou un vecteur selon la revendication 9 sont en dehors des chromosomes et/ou sont intégrés de manière stable dans le génome végétal.

**12.** Cellule végétale transgénique, plante complète et/ou leurs descendants selon l'une des revendications 10 ou 11, qui présentent une allénoxyde synthase selon la revendication 1 et/ou une divinyléther synthase selon la revendication 2, dont l'activité spécifique est supérieure à l'activité enzymatique spécifique endogène correspondante d'une cellule végétale.

**13.** Cellule végétale transgénique, plante complète et/ou leurs descendants selon l'une des revendications 10 à 12, **caractérisés en ce qu'**elles sont des plantes utiles ou leurs cellules, de préférence de la famille des solanacées ou des céréales et de façon particulièrement préférée des pommes de terre, de l'orge ou du blé.

**14.** Procédé d'augmentation de la résistance de plantes contre des agents pathogènes, **caractérisé en ce que** séparément ou en combinaison, une séquence de nucléotides selon l'une des revendications 4 à 7, et/ou une structure génétique selon la revendication 8 et/ou un vecteur selon la revendication 9 sont transférés sous forme réplicable dans des cellules végétales et des plantes complètes sont régénérées à partir des cellules végétales ainsi transformées.

**15.** Procédé selon la revendication 14, **caractérisé en ce que** les cellules végétales utilisées sont celles de plantes utiles qui appartiennent de préférence aux familles des solanacées ou des céréales et qui sont de façon particulièrement préférée la pomme de terre, l'orge ou le blé.

**16.** Utilisation d'au moins une séquence de nucléotides selon l'une des revendications 4 à 7 pour augmenter la résistance de cellules végétales, de plantes complètes et/ou de leurs descendants contre des agents pathogènes.

**17.** Utilisation d'au moins une enzyme selon l'une des revendications 1 à 6 pour augmenter la résistance de cellules végétales, de plantes complètes et/ou de leurs descendants contre des agents pathogènes.

**18.** Utilisation selon la revendication 17, dans laquelle l'activité spécifique d'au moins une enzyme dont l'activité est supérieure à l'activité enzymatique spécifique endogène correspondante rend les cellules végétales ou les plantes de 20 à 90 %, de préférence de 30 à 80 % et de façon particulièrement préférée de 40 à 70 % plus résistantes aux agents pathogènes.

**19.** Utilisation selon l'une des revendications 17 ou 18, dans laquelle au moins une enzyme augmente la résistance contre le champignon du mildiou et de préférence contre Blumeria graminis f. sp. hordei ou f. sp. tritici et/ou Phytophtora infestans.

Fig. 1

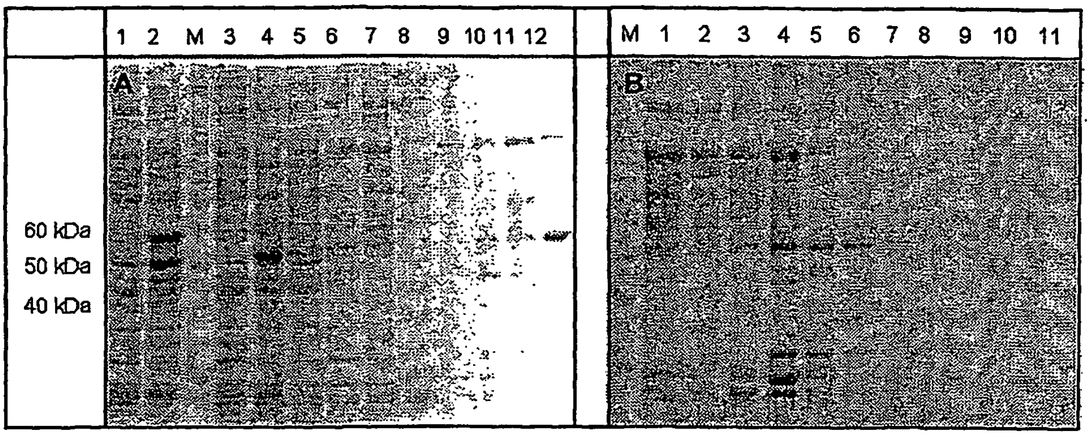

Fig. 2:

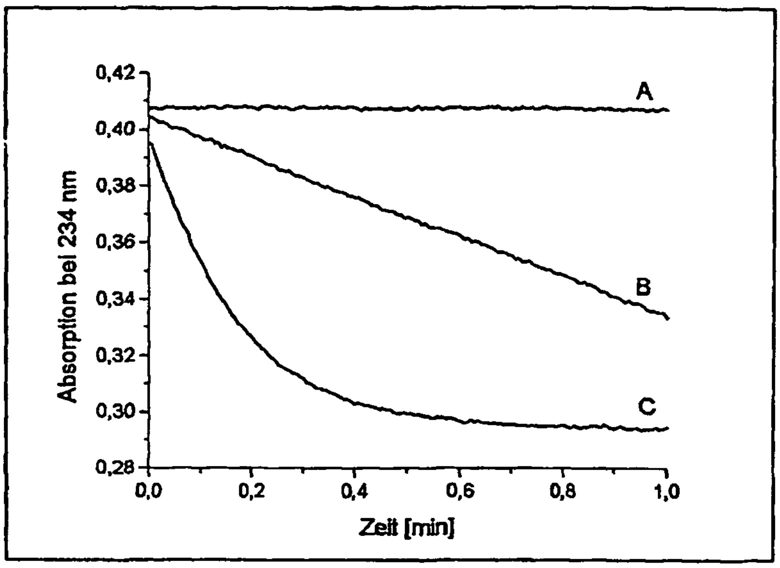
0,42
0,40
0,38
0,36
0,34
0,32
0,30
0,28
Absorption bei 234 nm
0,0
0,2
0,4
0,6
0,8
1,0
Zeit [min]
A
B
C

Fig. 3:

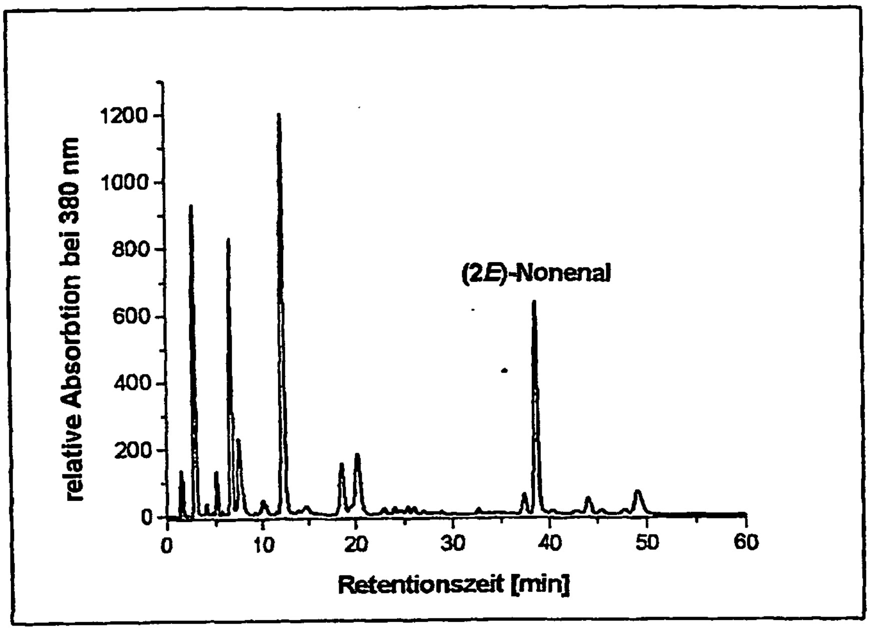
relative Absorbtion bei 380 nm
1200
1000
800
600
400
200
0
0
10
20
30
40
50
60
Retentionszeit [min]
(2E)-Nonenal

Fig. 4:

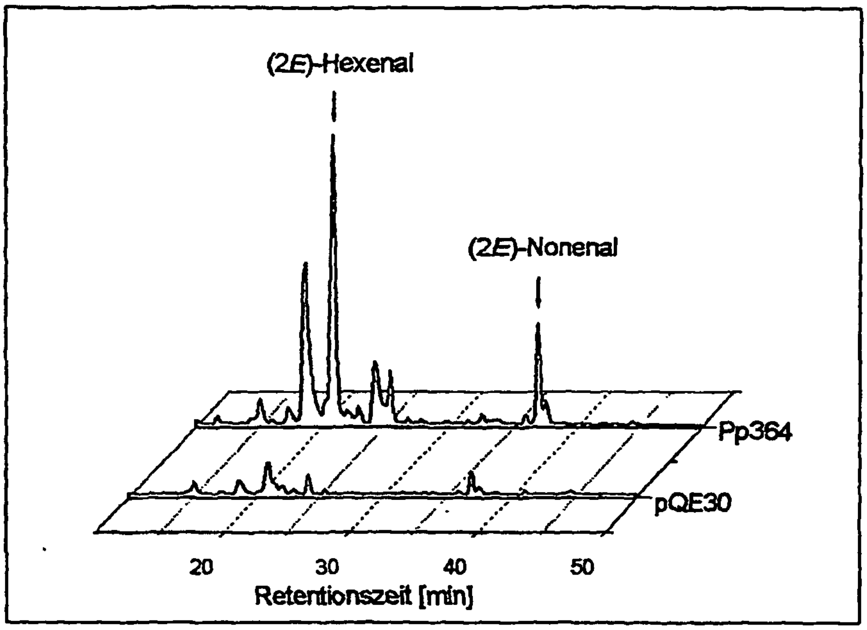
(2E)-Hexenal
(2E)-Nonenal
Pp364
pQE30
20
30
40
50
Retentionszeit [min]

Fig. 5:

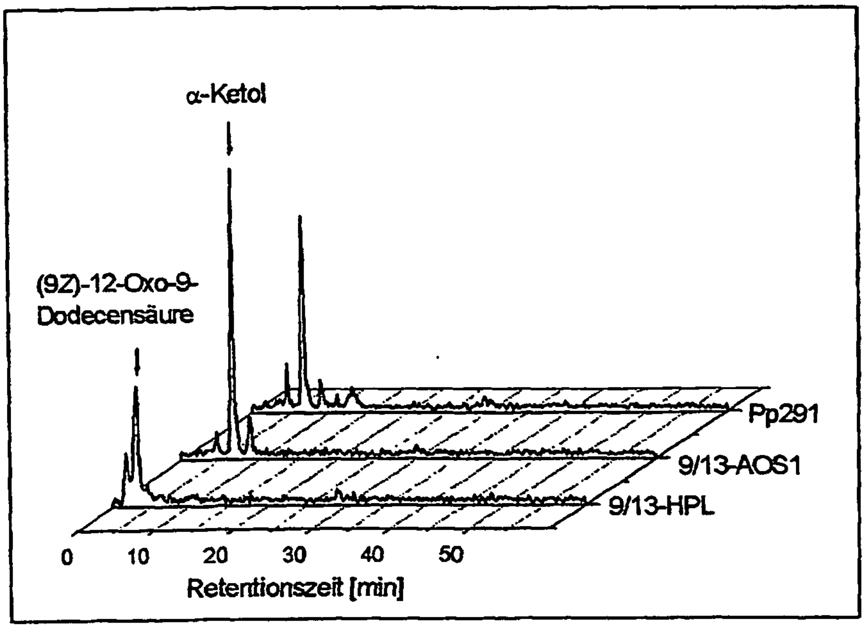
α-Ketol
(9Z)-12-Oxo-9-
Dodecensäure
Pp291
9/13-AOS1
9/13-HPL
0
10
20
30
40
50
Retentionszeit [min]

Fig. 6:

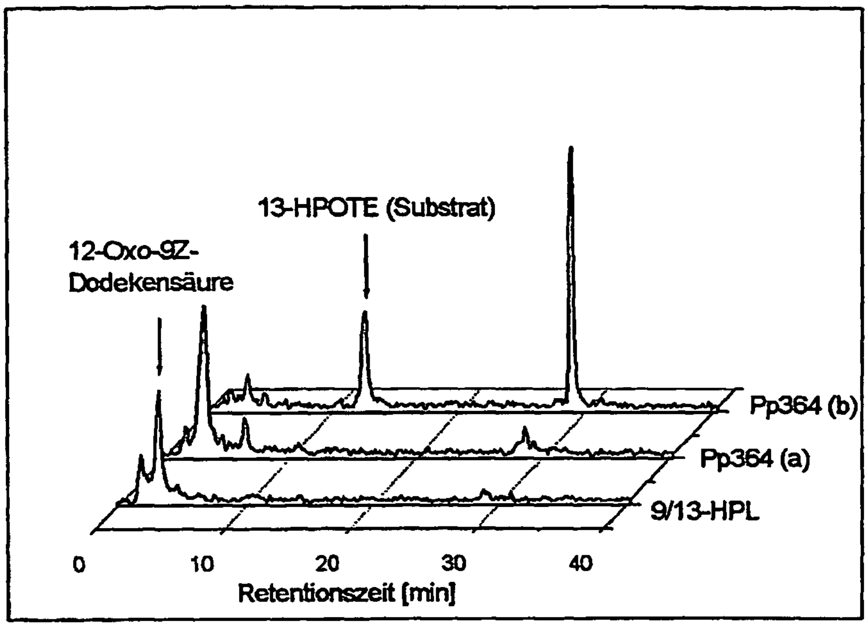
13-HPOTE (Substrat)
12-Oxo-9Z-
Dodekensäure
Pp364 (b)
Pp364 (a)
9/13-HPL
0
10
20
30
40
Retentionszeit [min]

Fig. 7:

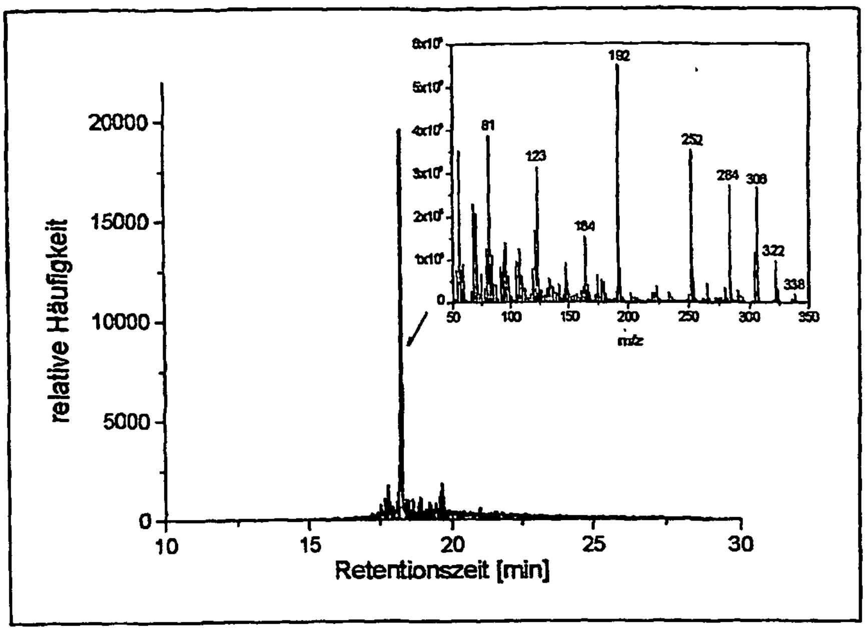
relative Häufigkeit
20000
15000
10000
5000
0
10
15
20
25
30
Retentionszeit [min]
192
81
123
252
284
308
164
322
338
50
100
150
200
250
300
350
m/z

Fig. 8:

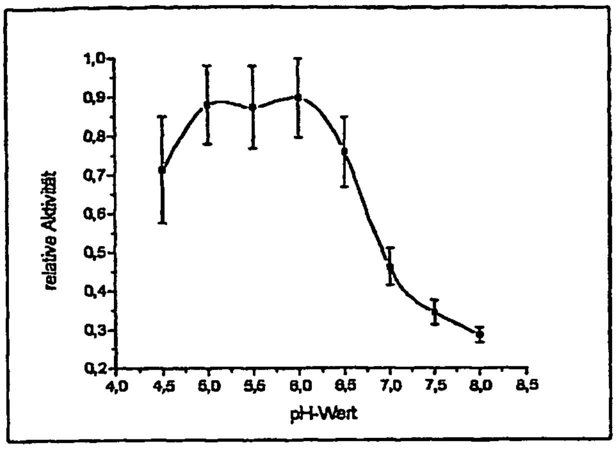
relative Aktivität
1,0
0,9
0,8
0,7
0,6
0,5
0,4
0,3
0,2
4,0 4,5 5,0 5,5 6,0 6,5 7,0 7,5 8,0 8,5
pH-Wert

Fig. 9:

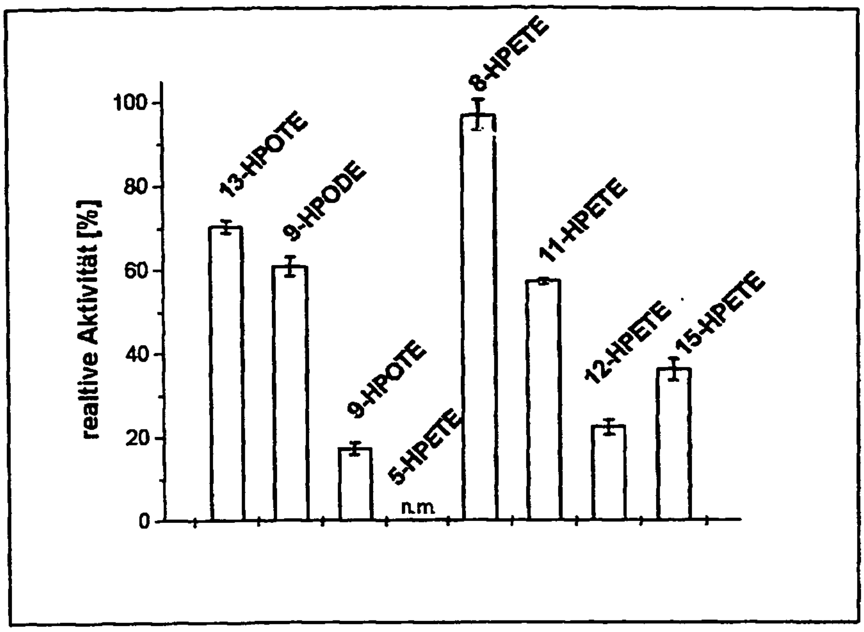
100
80
60
40
20
0
realtive Aktivität [%]
13-HPOTE
9-HPODE
9-HPOTE
5-HPETE
n.m.
8-HPETE
11-HPETE
12-HPETE
15-HPETE

SEQUENCE LISTING

<110> BASF Plant Schience GmbH

<120> CYP74-Enzyme und die sie kodierenden Nukleotidsequenzen sowie Verfahren zur Herstellung von pathogenresistenten Pflanzenzellen und deren Nachkommen

<130> 1
<160> 13
<170> Patentin version 3.1

<210> 1
<211> 1428
<212> DNA
<213> Physcomitrella patens

<220>
<221> CDS
<222> (1) .. (1428)
<223> Pp291 Allenoxid-Synthase

<400> 1

```
atg gca gtc cct tca tcc aag ctg ccg ttg aag gcg att cct gga gac      48
Met Ala Val Pro Ser Ser Lys Leu Pro Leu Lys Ala Ile Pro Gly Asp
1               5                   10                  15

tat gga gtc ccc tac ttc ggt gcc ata aag gat cga cta gac tac ttt      96
Tyr Gly Val Pro Tyr Phe Gly Ala Ile Lys Asp Arg Leu Asp Tyr Phe
            20                  25                  30

tgg ttg cag ggg gag gag cag ttt tac cga agc cgg atg gcc aag tac      144
Trp Leu Gln Gly Glu Glu Gln Phe Tyr Arg Ser Arg Met Ala Lys Tyr
        35                  40                  45

aat agc acg gtg ttt cgt gtc aac atg ccg cct ggc cct cca att tcc      192
Asn Ser Thr Val Phe Arg Val Asn Met Pro Pro Gly Pro Pro Ile Ser
    50                  55                  60

gaa cac cct caa gtc atc tgc ctc ttg gat cag aaa agc ttt cca att      240
Glu His Pro Gln Val Ile Cys Leu Leu Asp Gln Lys Ser Phe Pro Ile
65                  70                  75                  80

ctg ttc gac gtt agc aag gtt gag aaa aag gac gtg ttc aca gga aca      288
Leu Phe Asp Val Ser Lys Val Glu Lys Lys Asp Val Phe Thr Gly Thr
                85                  90                  95

tac atg ccg agt gtg agc ttc acc agc ggg tac cgc gtt tgc tcc tac      336
Tyr Met Pro Ser Val Ser Phe Thr Ser Gly Tyr Arg Val Cys Ser Tyr
            100                 105                 110

ttg gat ccc tct gag gaa cgc cac acg aag ctc aag caa tgg tgc ttt      384
Leu Asp Pro Ser Glu Glu Arg His Thr Lys Leu Lys Gln Trp Cys Phe
        115                 120                 125

gaa gtc att gcg atg aac ggg cgg aac ttt ctt ccc gag ttt cac aag      432
Glu Val Ile Ala Met Asn Gly Arg Asn Phe Leu Pro Glu Phe His Lys
    130                 135                 140
```

```
tcg att gaa gag tcg atg gtg ctc tgg gag acg agt ctg gcc aag ggc      480
Ser Ile Glu Glu Ser Met Val Leu Trp Glu Thr Ser Leu Ala Lys Gly
145                 150                 155                 160

gag aag act agc gta tcg gat gag gtg aaa cag ttc gcg ttt aat ttc      528
Glu Lys Thr Ser Val Ser Asp Glu Val Lys Gln Phe Ala Phe Asn Phe
                165                 170                 175

ctg atg cgc gct gta tgc cat cac gac ccc gct gcg cct gga gaa tac      576
Leu Met Arg Ala Val Cys His His Asp Pro Ala Ala Pro Gly Glu Tyr
            180                 185                 190

agc tta ggg cgt aat ggt ggc ccg tat gca acc gcc tgg gca aat ccc      624
Ser Leu Gly Arg Asn Gly Gly Pro Tyr Ala Thr Ala Trp Ala Asn Pro
        195                 200                 205

cag ctc gct ccg att gca gga cag acg ggt ctc ccc cat gtc gtg gag      672
Gln Leu Ala Pro Ile Ala Gly Gln Thr Gly Leu Pro His Val Val Glu
    210                 215                 220

gag ctc gtg tta cac acc gtc cca ctc ccc tct gcc ctg gtc aag aag      720
Glu Leu Val Leu His Thr Val Pro Leu Pro Ser Ala Leu Val Lys Lys
225                 230                 235                 240

aac tac gat gcc ctc tac aat ttc atc aaa aac tac gcc acc gag gcg      768
Asn Tyr Asp Ala Leu Tyr Asn Phe Ile Lys Asn Tyr Ala Thr Glu Ala
                245                 250                 255

ctg gat agg gct gaa gct atg ggc atc gag cgc aat gac gcc act gcc      816
Leu Asp Arg Ala Glu Ala Met Gly Ile Glu Arg Asn Asp Ala Thr Ala
            260                 265                 270

aac ctg ctg ttc ttc ctt tgc ttt aac gcc tac ggc gga ttc agc atc      864
Asn Leu Leu Phe Phe Leu Cys Phe Asn Ala Tyr Gly Gly Phe Ser Ile
        275                 280                 285

ttc ttc ccc ctc atc act atc ctc att tct tca tgc ggt ccg gag ctc      912
Phe Phe Pro Leu Ile Thr Ile Leu Ile Ser Ser Cys Gly Pro Glu Leu
    290                 295                 300

atg cac gat ctc cac gac gaa gtc acc aag gcc gtc gcc gcc aca gat      960
Met His Asp Leu His Asp Glu Val Thr Lys Ala Val Ala Ala Thr Asp
305                 310                 315                 320

ggg aaa gtc act ctt caa tcc atc gag aac atg cca ttg gtg aag tcc     1008
Gly Lys Val Thr Leu Gln Ser Ile Glu Asn Met Pro Leu Val Lys Ser
                325                 330                 335

gtc gtc tac gaa gct ttc cga ttc aag ccc cca gtg cca tac caa tac     1056
Val Val Tyr Glu Ala Phe Arg Phe Lys Pro Pro Val Pro Tyr Gln Tyr
            340                 345                 350

ggc aag gcc aag ttc gac ttc acc ata gag aac cac gaa aac tcc ttc     1104
Gly Lys Ala Lys Phe Asp Phe Thr Ile Glu Asn His Glu Asn Ser Phe
        355                 360                 365

gag gtc aag aag gga gaa atg ctg tat ggt tat caa cct atc gtg atg     1152
Glu Val Lys Lys Gly Glu Met Leu Tyr Gly Tyr Gln Pro Ile Val Met
    370                 375                 380

cac gac ccc aag gtc ttc tcg gac cca gat cag ttt cta cct cga cga     1200
His Asp Pro Lys Val Phe Ser Asp Pro Asp Gln Phe Leu Pro Arg Arg
385                 390                 395                 400
```

```
ttc atg ggc ccc gac ggc gag aag ctc atc aaa tac atc ttc tgg tcc     1248
Phe Met Gly Pro Asp Gly Glu Lys Leu Ile Lys Tyr Ile Phe Trp Ser
                405                 410                 415

aat ggt tac gag act gac gag ccg act acc gca aac aag cag tgc gcc     1296
Asn Gly Tyr Glu Thr Asp Glu Pro Thr Thr Ala Asn Lys Gln Cys Ala
            420                 425                 430

gga aag gac ttg gtg gtc aca atg gcg cga gca ttc gtc gca gaa atg     1344
Gly Lys Asp Leu Val Val Thr Met Ala Arg Ala Phe Val Ala Glu Met
        435                 440                 445

ttc ttg aga tat aaa gag tat acc ctg acc atg gag ggc gca gga aat     1392
Phe Leu Arg Tyr Lys Glu Tyr Thr Leu Thr Met Glu Gly Ala Gly Asn
    450                 455                 460

gcg acc aag gtt ttc ttt tcc gat ctc aaa aag tga                     1428
Ala Thr Lys Val Phe Phe Ser Asp Leu Lys Lys
465                 470                 475
```

<210> 2
<211> 475
<212> PRT
<213> Physcomitrella patens

<400> 2

Met Ala Val Pro Ser Ser Lys Leu Pro Leu Lys Ala Ile Pro Gly Asp
1 5 10 15

Tyr Gly Val Pro Tyr Phe Gly Ala Ile Lys Asp Arg Leu Asp Tyr Phe
20 25 30

Trp Leu Gln Gly Glu Glu Gln Phe Tyr Arg Ser Arg Met Ala Lys Tyr
35 40 45

Asn Ser Thr Val Phe Arg Val Asn Met Pro Pro Gly Pro Pro Ile Ser
50 55 60

Glu His Pro Gln Val Ile Cys Leu Leu Asp Gln Lys Ser Phe Pro Ile
65 70 75 80

Leu Phe Asp Val Ser Lys Val Glu Lys Lys Asp Val Phe Thr Gly Thr
85 90 95

Tyr Met Pro Ser Val Ser Phe Thr Ser Gly Tyr Arg Val Cys Ser Tyr
100 105 110

Leu Asp Pro Ser Glu Glu Arg His Thr Lys Leu Lys Gln Trp Cys Phe
115 120 125

Glu Val Ile Ala Met Asn Gly Arg Asn Phe Leu Pro Glu Phe His Lys
130 135 140

Ser Ile Glu Glu Ser Met Val Leu Trp Glu Thr Ser Leu Ala Lys Gly
145 150 155 160

Glu Lys Thr Ser Val Ser Asp Glu Val Lys Gln Phe Ala Phe Asn Phe
165 170 175

Leu Met Arg Ala Val Cys His His Asp Pro Ala Ala Pro Gly Glu Tyr
180 185 190

Ser Leu Gly Arg Asn Gly Gly Pro Tyr Ala Thr Ala Trp Ala Asn Pro
195 200 205

Gln Leu Ala Pro Ile Ala Gly Gln Thr Gly Leu Pro His Val Val Glu
210 215 220

Glu Leu Val Leu His Thr Val Pro Leu Pro Ser Ala Leu Val Lys Lys
225 230 235 240

Asn Tyr Asp Ala Leu Tyr Asn Phe Ile Lys Asn Tyr Ala Thr Glu Ala
245 250 255

Leu Asp Arg Ala Glu Ala Met Gly Ile Glu Arg Asn Asp Ala Thr Ala
260 265 270

Asn Leu Leu Phe Phe Leu Cys Phe Asn Ala Tyr Gly Gly Phe Ser Ile
275 280 285

Phe Phe Pro Leu Ile Thr Ile Leu Ile Ser Ser Cys Gly Pro Glu Leu
290 295 300

Met His Asp Leu His Asp Glu Val Thr Lys Ala Val Ala Ala Thr Asp
305 310 315 320

Gly Lys Val Thr Leu Gln Ser Ile Glu Asn Met Pro Leu Val Lys Ser
325 330 335

Val Val Tyr Glu Ala Phe Arg Phe Lys Pro Pro Val Pro Tyr Gln Tyr
340 345 350

Gly Lys Ala Lys Phe Asp Phe Thr Ile Glu Asn His Glu Asn Ser Phe
355 360 365

Glu Val Lys Lys Gly Glu Met Leu Tyr Gly Tyr Gln Pro Ile Val Met
370 375 380

His Asp Pro Lys Val Phe Ser Asp Pro Asp Gln Phe Leu Pro Arg Arg

385 390 395 400

Phe Met Gly Pro Asp Gly Glu Lys Leu Ile Lys Tyr Ile Phe Trp Ser
405 410 415

Asn Gly Tyr Glu Thr Asp Glu Pro Thr Thr Ala Asn Lys Gln Cys Ala
420 425 430

Gly Lys Asp Leu Val Val Thr Met Ala Arg Ala Phe Val Ala Glu Met
435 440 445

Phe Leu Arg Tyr Lys Glu Tyr Thr Leu Thr Met Glu Gly Ala Gly Asn
450 455 460

Ala Thr Lys Val Phe Phe Ser Asp Leu Lys Lys
465 470 475

<210> 3
<211> 1821
<212> DNA
<213> Physcomitrella patens

<220>
<221> CDS
<222> (25)..(1623)
<223> Pp364 Divinylether-Synthase

<400> 3

```
gcggaaaact ccgctccgat caat atg gat cgc act tta gtt ctg act tgc      51
                          Met Asp Arg Thr Leu Val Leu Thr Cys
                          1               5

act acg act tgc agc cac tcc gca ttc cgc cag tct gca ttg cct agc     99
Thr Thr Thr Cys Ser His Ser Ala Phe Arg Gln Ser Ala Leu Pro Ser
10                  15                  20                  25

aac acc agc ata tct gtg agg tta gga aca tgt agc gtt cgc aca cag    147
Asn Thr Ser Ile Ser Val Arg Leu Gly Thr Cys Ser Val Arg Thr Gln
                30                  35                  40

aag cgc cgt acg gtt gta gcc agt ctt ggg aac att gag acg aca tcg    195
Lys Arg Arg Thr Val Val Ala Ser Leu Gly Asn Ile Glu Thr Thr Ser
            45                  50                  55

aca tcg acc gtg ggg caa gag agc aat ctg ccc ctc cgt gaa atc ccc    243
Thr Ser Thr Val Gly Gln Glu Ser Asn Leu Pro Leu Arg Glu Ile Pro
        60                  65                  70

gga agc tac gga atc cct tat ttg tcg caa ttg ctc gac aga tgg acc    291
Gly Ser Tyr Gly Ile Pro Tyr Leu Ser Gln Leu Leu Asp Arg Trp Thr
    75                  80                  85

ttt ttt tac agg gaa ggc gaa ccg cag ttc tgg caa tca cga atg gcg    339
Phe Phe Tyr Arg Glu Gly Glu Pro Gln Phe Trp Gln Ser Arg Met Ala
90                  95                  100                 105

aag tat ggg agc acc gtg att cga tcc aac atg ccg cct ggt tgg ttt    387
```

```
Lys Tyr Gly Ser Thr Val Ile Arg Ser Asn Met Pro Pro Gly Trp Phe
                110                 115                 120

tgg acc gac tcc cgc tgc att atg ctt ctt gac cag aag agc tac ccc     435
Trp Thr Asp Ser Arg Cys Ile Met Leu Leu Asp Gln Lys Ser Tyr Pro
            125                 130                 135

acc gtc ttt gat tac gat aag gtg gat aag tac aaa gcc ttt gct ggg     483
Thr Val Phe Asp Tyr Asp Lys Val Asp Lys Tyr Lys Ala Phe Ala Gly
        140                 145                 150

acc atc atg cca agc acc gaa tac aat ggc ggg tat gag gtg tgt gcg     531
Thr Ile Met Pro Ser Thr Glu Tyr Asn Gly Gly Tyr Glu Val Cys Ala
    155                 160                 165

tac ctc gac gct tct gac aag aag cat gag cag ctc aaa ggc tat tgc     579
Tyr Leu Asp Ala Ser Asp Lys Lys His Glu Gln Leu Lys Gly Tyr Cys
170                 175                 180                 185

ttc gag ctt ctc aaa ttt tcc tcg tcg aaa tgg gca cgg gag ttt cac     627
Phe Glu Leu Leu Lys Phe Ser Ser Ser Lys Trp Ala Arg Glu Phe His
                190                 195                 200

acg gcc atc tca gag aca ttc aat cag tgg gaa ggc aaa ctt gca caa     675
Thr Ala Ile Ser Glu Thr Phe Asn Gln Trp Glu Gly Lys Leu Ala Gln
            205                 210                 215

aag acg cct gca tta att aac ccg acg ctt cct gaa tcg ctc ttt agt     723
Lys Thr Pro Ala Leu Ile Asn Pro Thr Leu Pro Glu Ser Leu Phe Ser
        220                 225                 230

ttt gtg atc aat gca ctg act acc gct aga ttc gac gac agt agc ata     771
Phe Val Ile Asn Ala Leu Thr Thr Ala Arg Phe Asp Asp Ser Ser Ile
    235                 240                 245

ccc gat gca gag aag cca gtc tgc ggg gat ttg caa aaa tgg gcg gga     819
Pro Asp Ala Glu Lys Pro Val Cys Gly Asp Leu Gln Lys Trp Ala Gly
250                 255                 260                 265

ttc cag ctg atg ccc gta atc aga acc ggg gca cct atc tac att gaa     867
Phe Gln Leu Met Pro Val Ile Arg Thr Gly Ala Pro Ile Tyr Ile Glu
                270                 275                 280

gag atg ctc cac gtt gct ccc atc cct gca agc cta act aaa ggg ggc     915
Glu Met Leu His Val Ala Pro Ile Pro Ala Ser Leu Thr Lys Gly Gly
            285                 290                 295

tat gac aaa atg gtg gtg ttt ctt caa aag tat gcg gct gaa acg cta     963
Tyr Asp Lys Met Val Val Phe Leu Gln Lys Tyr Ala Ala Glu Thr Leu
        300                 305                 310

tcc atc gca gag aag ttt ggg ttg tct cag gac gag gcg gtt cac aac    1011
Ser Ile Ala Glu Lys Phe Gly Leu Ser Gln Asp Glu Ala Val His Asn
    315                 320                 325

ttg atc ttc ttc cta atc ttg aac gct cat ggc gga ttc tgc cgg ttc    1059
Leu Ile Phe Phe Leu Ile Leu Asn Ala His Gly Gly Phe Cys Arg Phe
330                 335                 340                 345

ctt cca gtg atc ctt cgg gaa gta gcc aag aat ggc caa ctg caa gct    1107
Leu Pro Val Ile Leu Arg Glu Val Ala Lys Asn Gly Gln Leu Gln Ala
                350                 355                 360
```

```
gat ttg cga gag gaa gtg cgg gcc gca gtg aaa gcc agc gga tcg gac       1155
Asp Leu Arg Glu Glu Val Arg Ala Ala Val Lys Ala Ser Gly Ser Asp
        365                 370                 375

caa gtg acc atg aag gcc gtg atg aat gac atg cct ctg gtg gca tcg       1203
Gln Val Thr Met Lys Ala Val Met Asn Asp Met Pro Leu Val Ala Ser
    380                 385                 390

aca gta ttc gag gcg ctc cgc ttc gac ccc ccg gtg cca ttt cag tac       1251
Thr Val Phe Glu Ala Leu Arg Phe Asp Pro Pro Val Pro Phe Gln Tyr
395                 400                 405

gcc aga gcg aag aag gac ttc atc atc gaa tcc cac gac gcg aga tac       1299
Ala Arg Ala Lys Lys Asp Phe Ile Ile Glu Ser His Asp Ala Arg Tyr
410                 415                 420                 425

caa ata aaa acc ggc gac ttc ctc ggc ggc gtg aac tac atg gtc tcc       1347
Gln Ile Lys Thr Gly Asp Phe Leu Gly Gly Val Asn Tyr Met Val Ser
                430                 435                 440

cgc gac ccg aag gtg ttc acc gac agg ccc aac gag ttc aac gcg cgg       1395
Arg Asp Pro Lys Val Phe Thr Asp Arg Pro Asn Glu Phe Asn Ala Arg
            445                 450                 455

cgg ttc atg gga ccg gag ggg gac aag ctg ctt gca cat ttg gtg tgg       1443
Arg Phe Met Gly Pro Glu Gly Asp Lys Leu Leu Ala His Leu Val Trp
        460                 465                 470

tcg aac ggc cgg caa act gat gaa acc acg gtg tac aca aag cag tgt       1491
Ser Asn Gly Arg Gln Thr Asp Glu Thr Thr Val Tyr Thr Lys Gln Cys
    475                 480                 485

gcg ggg aag gag att gtg ccg ctc aca ggg cgc ctt ctt ctg gcg gag       1539
Ala Gly Lys Glu Ile Val Pro Leu Thr Gly Arg Leu Leu Leu Ala Glu
490                 495                 500                 505

ctt ttc atg cgc ttc gat tcc ttc aac atc gaa ggc ctc gaa atg gag       1587
Leu Phe Met Arg Phe Asp Ser Phe Asn Ile Glu Gly Leu Glu Met Glu
                510                 515                 520

gca acc ttc act tca ctg acg ccg cga tca gat tga agctatagct            1633
Ala Thr Phe Thr Ser Leu Thr Pro Arg Ser Asp
            525                 530

tgtaaaacac ccaccccacg ttgtgagatt attagtacca cgtacatcag tagttcacga     1693

gactcatatt ctgatccatc atcgcctgga tgtcgaaact gactatatgt agtatactcg     1753

actttgtatg ccaaaaacac attttcaatt tgtctaatcg gccctgtttc cacttcaaaa     1813

aaaaaaaa                                                              1821
```

<210> 4
<211> 532
<212> PRT
<213> Physcomitrella patens

<400> 4

Met Asp Arg Thr Leu Val Leu Thr Cys Thr Thr Thr Cys Ser His Ser
1 5 10 15

Ala Phe Arg Gln Ser Ala Leu Pro Ser Asn Thr Ser Ile Ser Val Arg
20 25 30

Leu Gly Thr Cys Ser Val Arg Thr Gln Lys Arg Arg Thr Val Val Ala
35 40 45

Ser Leu Gly Asn Ile Glu Thr Thr Ser Thr Ser Thr Val Gly Gln Glu
50 55 60

Ser Asn Leu Pro Leu Arg Glu Ile Pro Gly Ser Tyr Gly Ile Pro Tyr
65 70 75 80

Leu Ser Gln Leu Leu Asp Arg Trp Thr Phe Phe Tyr Arg Glu Gly Glu
85 90 95

Pro Gln Phe Trp Gln Ser Arg Met Ala Lys Tyr Gly Ser Thr Val Ile
100 105 110

Arg Ser Asn Met Pro Pro Gly Trp Phe Trp Thr Asp Ser Arg Cys Ile
115 120 125

Met Leu Leu Asp Gln Lys Ser Tyr Pro Thr Val Phe Asp Tyr Asp Lys
130 135 140

Val Asp Lys Tyr Lys Ala Phe Ala Gly Thr Ile Met Pro Ser Thr Glu
145 150 155 160

Tyr Asn Gly Gly Tyr Glu Val Cys Ala Tyr Leu Asp Ala Ser Asp Lys
165 170 175

Lys His Glu Gln Leu Lys Gly Tyr Cys Phe Glu Leu Leu Lys Phe Ser
180 185 190

Ser Ser Lys Trp Ala Arg Glu Phe His Thr Ala Ile Ser Glu Thr Phe
195 200 205

Asn Gln Trp Glu Gly Lys Leu Ala Gln Lys Thr Pro Ala Leu Ile Asn
210 215 220

Pro Thr Leu Pro Glu Ser Leu Phe Ser Phe Val Ile Asn Ala Leu Thr
225 230 235 240

Thr Ala Arg Phe Asp Asp Ser Ser Ile Pro Asp Ala Glu Lys Pro Val
245 250 255

Cys Gly Asp Leu Gln Lys Trp Ala Gly Phe Gln Leu Met Pro Val Ile
260 265 270

Arg Thr Gly Ala Pro Ile Tyr Ile Glu Glu Met Leu His Val Ala Pro
275 280 285

Ile Pro Ala Ser Leu Thr Lys Gly Gly Tyr Asp Lys Met Val Val Phe
290 295 300

Leu Gln Lys Tyr Ala Ala Glu Thr Leu Ser Ile Ala Glu Lys Phe Gly
305 310 315 320

Leu Ser Gln Asp Glu Ala Val His Asn Leu Ile Phe Phe Leu Ile Leu
325 330 335

Asn Ala His Gly Gly Phe Cys Arg Phe Leu Pro Val Ile Leu Arg Glu
340 345 350

Val Ala Lys Asn Gly Gln Leu Gln Ala Asp Leu Arg Glu Glu Val Arg
355 360 365

Ala Ala Val Lys Ala Ser Gly Ser Asp Gln Val Thr Met Lys Ala Val
370 375 380

Met Asn Asp Met Pro Leu Val Ala Ser Thr Val Phe Glu Ala Leu Arg
385 390 395 400

Phe Asp Pro Pro Val Pro Phe Gln Tyr Ala Arg Ala Lys Lys Asp Phe
405 410 415

Ile Ile Glu Ser His Asp Ala Arg Tyr Gln Ile Lys Thr Gly Asp Phe
420 425 430

Leu Gly Gly Val Asn Tyr Met Val Ser Arg Asp Pro Lys Val Phe Thr
435 440 445

Asp Arg Pro Asn Glu Phe Asn Ala Arg Arg Phe Met Gly Pro Glu Gly
450 455 460

Asp Lys Leu Leu Ala His Leu Val Trp Ser Asn Gly Arg Gln Thr Asp
465 470 475 480

Glu Thr Thr Val Tyr Thr Lys Gln Cys Ala Gly Lys Glu Ile Val Pro
485 490 495

Leu Thr Gly Arg Leu Leu Leu Ala Glu Leu Phe Met Arg Phe Asp Ser
500 505 510

Phe Asn Ile Glu Gly Leu Glu Met Glu Ala Thr Phe Thr Ser Leu Thr

515 520 525

Pro Arg Ser Asp
530

<210> 5
<211> 30
<212> DNA
<213> artificial

<220>
<221> T7lang, RACE-primer
<222> (1).. (30)
<223>

<400> 5

gtaatacgac tcactatagg gcgaattggg 30

<210> 6
<211> 19
<212> DNA
<213> artificial

<220>
<221> M13rev; RACE-primer
<222> (1).. (19)
<223>

<400> 6

ggaaacagct atgaccatg 19

<210> 7
<211> 23
<212> DNA
<213> artificial

<220>
<221> PP291AOS5R; genspezifische_RACE-primer
<222> (1) ..(23)
<223>

<400> 7

tcacctcatc cgatacgcta gtc 23

<210> 8
<211> 22
<212 > DNA.
<213> artificial

<220>
<221> Pp364AOS5R; genspezifische_RACE-primer
<222> (1).. (22)
<223>

<400> 8

gtcgatgtcg tctcaatgtt cc 22

<210> 9
<211> 23
<212> DNA
<213> artificial

<220>
<221> Pp364AOS5R2; genspezifische_RACE-primer
<222> (1) .. (23)
<223>

<400> 9

ccattcgtga ttgccagaac tgc 23

<210> 10
<211> 31
<212> DNA
<213> artificial

<220>
<221> Pp291/5'-SphI; Expressions-primer
<222> (1)..(31)
<223>

<400> 10

aaagcatgca tggcagtccc ttcatccaag c 31

<210> 11
<211> 45
<212> DNA
<213> artificial

<220>
<221> Pp291/3' PstI; Expressions-primer
<222> (1)..(45)
<223>

<400> 11

aaactgcagt cactttttga gatcggaaaa gaaaaccttg gtcgc 45

<210> 12

<211> 30
<212> DNA
<213> artificial

<220>
<221> Pp364/5'BamHI; Expressions-primer
<222> (1).. (30)
<223>

<400> 12

ggatcccgta cggttgtagc cagtcttggg 30

<210> 13
<211> 29
<212> DNA
<213> artificial

<220>
<221> pp364/3'HindIII; Expressions-primer
<222> (1)..(29)
<223>

<400> 13

aagctttcaa tctgatcgcg gcgtcagtg 29